(19) Europäisches Patentamt
European Patent Office
Office européen des brevets

(11) Publication number: **0 508 004 A1**

# (12) EUROPEAN PATENT APPLICATION

(21) Application number: **91303079.7**

(22) Date of filing: **08.04.91**

(51) Int. Cl.[5]: **C07C 317/44**, A61K 31/16, C07C 323/62

(43) Date of publication of application:
**14.10.92 Bulletin 92/42**

(84) Designated Contracting States:
**AT BE CH DE ES FR GB IT LI NL SE**

(71) Applicant: **Kissei Pharmaceutical Co., Ltd.**
**No. 19-48 Yoshino**
**Matsumoto-City Nagano-Pref. 399-65(JP)**

(72) Inventor: **Kitazawa, Makio**
**No. 945, Oaza Yoshikawa Hirata**
**Matsumoto-shi, Nagano-ken(JP)**
Inventor: **Akahane, Masuo**
**No. B-1 Village-Sato, No. 3249-1, Satoyamabe**
**Matsumoto-shi, Nagano-ken(JP)**
Inventor: **Nakano, Yasushi**
**No. 4997-1, Soga**
**Shiojiri-shi, Nagano-ken(JP)**
Inventor: **Tsubaki, Atsushi**
**No. 1-4-8, Sasabe**
**Matsumoto-shi, Nagano-ken(JP)**
Inventor: **Sato, Kazuaki, c/o Kissei Daini-Seiyuryo**
**No. 1-2-34, Nomizo Mokko**
**Matsumoto-shi, Nagano-ken(JP)**
Inventor: **Ban, Masaaki, c/o Kissei Daini-Seiyuryo**
**No. 1-2-34, Nomizo Mokko**
**Matsumoto-shi, Nagano-ken(JP)**
Inventor: **Kobayashi, Michihiro**
**No. 3158, Oaza Nakagawate, Akashina-machi**
**Higashichikuma-gun, Nagano-ken(JP)**

(74) Representative: **Allard, Susan Joyce et al**
**BOULT, WADE & TENNANT, 27 Furnival Street**
**London EC4A 1PO(GB)**

(54) **Phenylsulfonylalkanoic acid compounds.**

(57) The present invention provides a novel class of phenylsulfonylalkanoic acid compounds, for example, a structure corresponding to the formula:

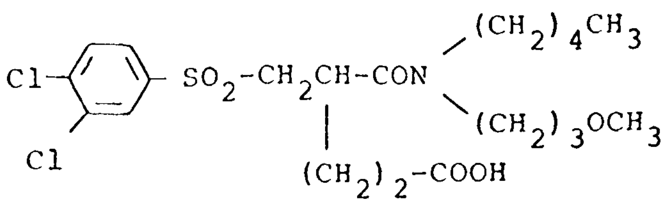

or a pharmaceutically acceptable salt thereof.

Phenylsulfonylalkanoic acid compounds of the present invention are useful for prevention or treatment of irritable bowel syndrome, biliary dyskinesia and acute pancreatitis.

EP 0 508 004 A1

The present invention relates to phenylsulfonylalkanoic acid compounds which are useful therapeutic agents.

Analogs of Proglumide represented by the formula:

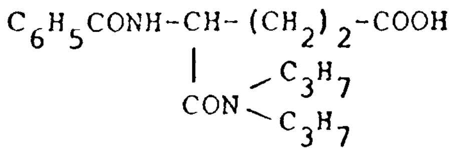

(II)

are reported in patent applications which include Japanese Patent Application (OPI) Nos. 44855/86, 181246/87, 27468/88, 165352/88 and 201156/88 (The term "OPI" used herein refers to an unexamined Japanese Patent Application), EP(A1)0308885, EP(A2)0272228, WO87/03869, WO88/05774 and WO89/02431. The compounds described in these patent applications are derived from amino acid compounds such as glutamic acid or aspartic acid.

Cholecystokinin, hereinafter referred to as CCK, is a typical gastrointestinal hormone with activity that stimulates exocrine pancreatic secretion and contracts the gallbladder.

The present invention provides novel phenylsulfonylalkanoic acid compounds which exhibit strong antagonistic activities toward CCK receptors, and thus they are useful as therapeutic agents for the prevention and treatment of irritable bowel syndrome, biliary dyskinesia and acute pancreatitis.

In accordance with the present invention there is provided phenylsulfonylalkanoic acid compounds represented by the formula:

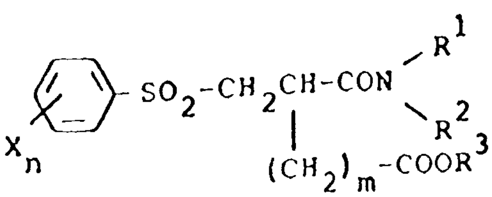

(I)

wherein $R^1$ represents an alkyl group having 1 to 10 carbon atoms, an alkoxyalkyl group having 3 to 7 carbon atoms or an aralkyl group having 7 to 12 carbon atoms; $R^2$ represents an alkyl group having 1 to 10 carbon atoms or an aralkyl group having 7 to 12 carbon atoms; $R^3$ represents a hydrogen atom or an alkyl group having 1 to 4 carbon atoms; X represents a halogen atom, an alkyl group having 1 to 3 carbon atoms, a nitro group or an acetyl group; m is an integer of from 1 to 3; n is 1 or 2; and pharmaceutically acceptable salts thereof.

The present invention includes pharmaceutical compositions containing a phenysulfonylalkanoic acid compound as an active ingredient.

A particularly preferred compound of the invention is a compound represented by the formula:

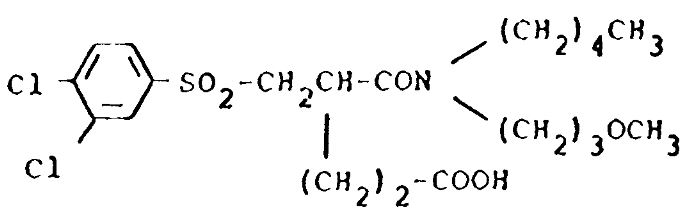

The invention also includes a compound represented by the formula:

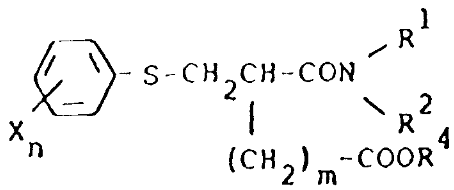

(III)

in which $R^1$, $R^2$, X, m and n are as previously defined and $R^4$ is an alkyl group having 1 to 4 carbon atoms.

The invention further includes a compound represented by the formula:

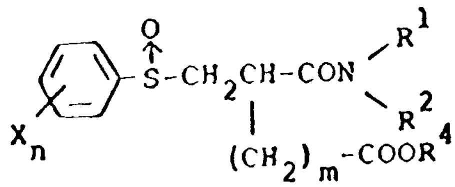

(IV)

in which $R^1$, $R^2$, X, m and n, and $R^4$ are as previously defined.

The phenylsulfonylalkanoic acid compounds of the present invention exhibit antagonistic activity toward CCK receptors. They competitively inhibit CCK-8 from binding to CCK receptors, and thus exhibit inhibitory activities against gallbladder contraction and amylase secretion by CCK-8.

Thus, they are useful as therapeutic agents for the prevention or treatment of irritable bowel syndrome, biliary dyskinesia and acute pancreatitis.

The term "alkyl" in the present invention refers to a straight or branched alkyl group having 1 to 10 carbon atoms, such as methyl or isobutyl.

The term "alkoxyalkyl" in the present invention refers to a straight or branched alkoxyalkyl group having 3 to 7 carbon atoms, such as methoxyethyl or isopropoxyethyl.

The term "aralkyl" in the present invention refers to a straight or branched alkyl group having phenyl group as a substituent and it has 7 to 12 carbon atoms, such as benzyl, phenethyl or phenylpropyl.

The novel phenylsulfonylalkanoic acid compounds of forumla (I) can be prepared by oxidizing phenylthioalkanoic acid compounds represented by the forumla:

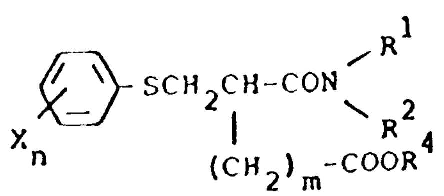

(III)

wherein $R^1$, $R^2$, X, m and n are as previously defined;
and $R^4$ represents an alkyl group having 1 to 4 carbon atoms or phenylsulfinylalkanoic acid compounds represented by the formula:

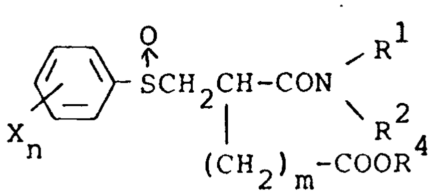

(IV)

wherein $R^1$, $R^2$, $R^4$, X, m and n are as previously defined.

The corresponding carboxylic acid compounds can be prepared by hydrolysis of the ester products.

The present invention phenylsulfonylalkanoic acid formula (I) compounds have one asymmetric carbon

atom in the structure, and consequently exist in the form of two optical isomers. The configuration of substituents on the asymmetric carbon atom is not limited, and S-configuration, R-configuration or a mixture of S- and R-configurations can be employed in the present invention.

Phenylsulfonylalkanoic acid formula (I) compounds in which $R^1$ is an alkyl group or an alkoxyalkyl group, $R^2$ is an alkyl group, $R^3$ is a hydrogen atom, X is a halogen atom, m is 2 and n is 2 are preferred structures in the practice of the present invention embodiments.

Particularly preferred invention phenylsulfonylalkanoic acid compounds include 5-(3,4-dichlorophenylsulfonyl)-4-[N-(3-methoxypropyl)-N-pentylcarbamoyl]-pentanoic acid, its optical isomers and the pharmaceutically acceptable salts thereof.

The compounds of formula (III) used as starting materials in the present invention are novel compounds and can be prepared by reacting thiophenol compounds represented by the formula:

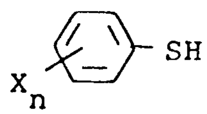

(V)

where X and n are as previously defined with compounds represented by the formula:

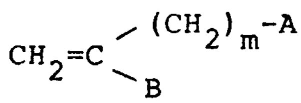

(VI)

where (1) A and B are the same and are a cyano group or an alkoxycarbonyl group having 2 to 5 carbon atoms, or (2) A represents an alkoxycarbonyl group having 2 to 5 carbon atoms and B represents a carboxy group or alkali metal salts thereof, and m is as previously defined, in the presence of a Lewis-base or Lewis-acid to form a product represented by the formula:

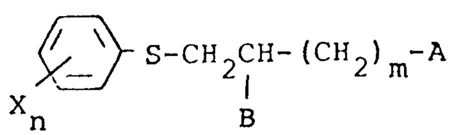

(VII)

wherein A, B, X, m, and n are as previously defined.

As a further alternative, hydrolyzing and then mono-esterifying the product provides a compound represented by the formula:

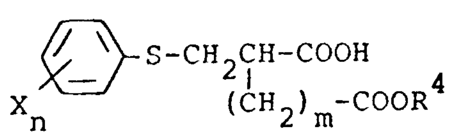

(VIII)

where $R^4$, X, m and n are as previously defined. The compound of formula (VIII) or a reactive functional derivative thereof subsequently can be reacted with an amino compound represented by the formula:

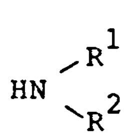

(IX)

wherein $R^1$ and $R^2$ are as previously defined, to provide a selected compound of formula (III).

The compounds of formula (IV) used too as starting materials in the present invention are novel compounds and can be prepared by oxidizing phenylthioalkanoic acid compounds (III).

The compounds of formula (IV) used too as starting materials in the present invention are novel compounds and can be prepared by oxidizing phenylthioalkanoic acid compounds (III).

A synthesis procedure of the present invention can be conducted by dissolving a compound of formula (III) in a suitable organic solvent such as dichloromethane, then adding a molar excess(e.q., a 2.5 molar ratio) of an oxidizing agent such as m-chloroperbenzoic acid to the solution with cooling, stirring the reaction mixture for 2-3 hours viith cooling or at room temperature, and working up the reaction mixture in accordance with standard methods to yield compounds of formula (I) wherein $R^3$ is a lower alkyl group. Hydrolysis of an ester product provides a compound of formula (I) in which $R^3$ is a hydrogen atom.

The compounds of formula (I) in which $R^3$ is a hydrogen atom can be converted to pharmaceutically acceptable salts using standard procedures. Present invention salts include inorganic base salts such as a sodium salt or a calcium salt, and organic salts which are formed with bases such as morpholine, piperidine, arginine, and the like.

Salt compounds of the present invention exhibit a similar antagonistic activity toward CCK receptors as the corresponding free carboxylic acid compounds, and thus they are useful as therapeutic agents for the prevention or treatment of irritable bowel syndrome, biliary dyskinesia and acute pancreatitis.

When the phenylsulfonylalkanoic acid compounds of formula (I) of the present invention or the pharmaceutically acceptable salts thereof are employed therapeutically, they can be administrated in various dosage forms depending upon the intended therapy. Typical dosage forms which can be used are tablets, pills, powders, granules, capsules and injectable preparations. These pharmaceutical compositions can be formulated in accordance with standard molding procedures.

The dosage of the phenylsulfonylalkanoic acid compounds of the present invention may be in the range from about 1 to 1000 mg per adult human by oral administration per day, or from about 0.1 to 100 mg per adult human by parenteral administration per day in multiple doses depending upon the type of disease, the severity of the condition to be treated, and the like.

The present invention is further illustrated in more detail by way of the following Examples and Reference Examples. The melting points of the products obtained are uncorrected.

Reference example 1

3-(3,4-Dichlorophenylthio)-2-(2-methoxycarbonylethyl)propionic acid

A mixture of 3,4-dichlorobenzenethiol (3.00 ml), methyleneglutaronitrile (2.57 ml), ethanol (25 ml), and triton B (40% methanol solution, 10 drops) was refluxed for 4 hours. After the reaction mixture was concentrated in vacuo, the resulting residue was dissolved in chloroform, washed with water, and dried over $MgSO_4$. The solvent was evaporated at reduced pressure, and the residue was recrystallized from diethyl ether-hexane to give 6.14 g of 2-(3,4-dichlorophenylthiomethyl)glutaronitrile.
Melting point: 53-55 ° C

| Elemental Analysis (for $C_{12}H_{10}Cl_2N_2S$): | | | |
|---|---|---|---|
| | C% | H% | N% |
| Calcd. | 50.54 | 3.53 | 9.82 |
| Found | 50.35 | 3.39 | 9.87 |

IR (KBr): $\nu_{CN}$ 2240 $cm^{-1}$
NMR ($CDCl_3$)
δ: 1.95-2.25(2H, m), 2.45-2.75(2H, m), 2.85-2.95(1H, m), 3.10(1H, dd, J = 6.6, 14.3Hz), 3.22(1H, dd, J = 7.1, 14.3Hz), 7.28(1H, dd, J = 1.7, 8.2Hz), 7.43(1H, d, J = 8.2Hz), 7.54(1H, d, J = 1.7Hz)

To a solution of 2-(3,4-dichlorophenylthiomethyl)glutaronitrile (5.40 g) in acetic acid (36 ml) was added a concentrated hydrochloric acid (36 ml), and the mixture was refluxed for 20 hours. After the reaction mixture was concentrated in vacuo, diethyl ether was added, and the precipitates formed were filtered off. The filtrate was washed with water, and shaken with a saturated sodium bicarbonate solution. The aqueous layer was acidified with a concentrated hydrochloric acid, and extracted with diethyl ether. The organic layer was washed with water, dried over $MgSO_4$, and evaporated at reduced pressure. The residue was recrystallized from diethyl ether-hexane to give 4.44 g of 2-(3,4-dichlorophenylthiomethyl)glutaric acid.

Melting point: 112-114 °C

| Elemental Analysis (for $C_{12}H_{12}Cl_2O_4S$): | | |
|---|---|---|
| | C% | H% |
| Calcd.<br>Found | 44.60<br>44.35 | 3.74<br>3.66 |

IR (KBr): $\nu_{C=O}$ 1710 $cm^{-1}$
NMR ($CDCl_3$)
δ: 1.9-2.1(2H, m), 2.25-2.5(2H, m), 2.55-2.7(1H, m), 3.01(1H, dd, J=6.0, 13.2Hz), 3.23(1H, dd, J=7.7, 13.2Hz), 7.20(1H, dd, J=2.2, 8.2Hz), 7.35(1H, d, J=8.2Hz), 7.44(1H, d, J=2.2Hz)

A mixture of 2-(3,4-dichlorophenylthiomethyl)glutaric acid (3.00 g), methanol (30 ml), and p-toluenesulfonic acid (0.09 g) was stirred at 40 °C for 2.5 hours. The reaction mixture was concentrated in vacuo, and the resulting residue was purified by flash column chromatography on silica eluting with chloroform/ethanol (10:1) to give 2.92 g of 3-(3,4-dichlorophenylthio)-2-(2-methoxycarbonylethyl)propionic acid as oil.
IR (neat): $\nu_{C=O}$ 1740, 1710 $cm^{-1}$
NMR ($CDCl_3$)
δ: 1.95-2.1(2H, m), 2.3-2.5(2H, m), 2.6-2.75(1H, m), 3.01(1H, dd, J=6.0, 13.2Hz), 3.23(1H, dd, J=7.7, 13.2Hz), 3.67(3H, s), 7.19(1H, dd, J=2.2, 8.2Hz), 7.36(1H, d, J=8.2Hz), 7.45(1H, d, J=2.2Hz)

Reference example 2

The compounds in the table were prepared in a similar manner to that described in reference example 1 (all compounds were oils).

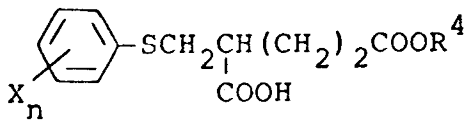

| $X_n$ | $R^4$ | IR ($cm^{-1}$) | NMR (δ, $CDCl_3$) |
|---|---|---|---|
| 3,5-dichloro | methyl | 1730, 1705 (C=O) | 1.95-2.1(2H, m), 2.3-2.5(2H, m), 2.6-2.8(1H, m), 3.04(1H, dd, J=6.0, 13.2Hz), 3.25(1H, dd, J=7.7, 13.2Hz), 3.67(3H, s), 7.1-7.25(3H, m) |
| 4-chloro | methyl | 1730, 1705 (C=O) | 1.95-2.1(2H, m), 2.3-2.5(2H, m), 2.6-2.75(1H, m), 2.99 (1H, dd, J=6.0, 13.2Hz), 3.21(1H, dd, J=7.7, 13.2Hz), 3.66(3H, s), 7.2-7.35(4H, m) |
| 3-chloro | methyl | 1730, 1710 (C=O) | 1.95-2.15(2H, m), 2.3-2.5(2H, m), 2.6-2.75(1H, m), 3.02 (1H, dd, J=6.0, 13.7Hz), 3.25(1H, dd, J=7.7, 13.7Hz), 3.66(3H, s), 7.15-7.3(3H, m), 7.35(1H, t, J=1.1Hz) |
| 3-bromo | methyl | 1735, 1710 (C=O) | 1.95-2.1(2H, m), 2.3-2.5(2H, m), 2.6-2.75(1H, m), 3.02 (1H, dd, J=6.0, 13.7Hz), 3.24(1H, dd, J=7.7, 13.7Hz), 3.67(3H, s), 7.16(1H, t, J=7.7Hz), 7.25-7.4(2H, m), 7.51(1H, t, J=1.7Hz) |
| 3,4-dichloro | ethyl | 1730, 1710 (C=O) | 1.24(3H, t, J=7.1Hz), 1.95-2.1(2H, m), 2.25-2.5(2H, m), 2.6-2.75(1H, m), 3.02(1H, dd, J=6.0, 13.2Hz), 3.23(1H, dd, J=7.7, 13.2Hz), 4.12(2H, q, J=7.1Hz), 7.20(1H, dd, J=2.2, 8.2Hz), 7.36(1H, d, J=8.2Hz), 7.45(1H, d, J=2.2Hz) |

T-1

Reference example 3

(+)-3-(3,4-Dichlorophenylthio)-2-(2-methoxycarbonylethyl)propionic acid and (-)-3-(3,4-dichlorophenylthio)-2-(2-methoxycarbonylethyl)propionic acid

A mixture of (±)-3-(3,4-dichlorophenylthio)-2-(2-methoxycarbonylethyl)propionic acid (144.1 g), quinine (161.0 g), and 2-propanol (400 ml) was heated to solution, and about 250 ml of 2-propanol was evaporated at reduced pressure. The residual solution was allowed to stand at room temperature. The precipitated crystals were collected, and further recrystallized three times from 2-propanol to give 113.0 g of the salt formed from (+)-3-(3,4-dichlorophenylthio)-2-(2-methoxycarbonylethyl)propionic acid and quinine. To this salt (0.64 g) was added a 2N hydrochloric acid (20 ml), and a separated oil was extracted with ethyl acetate. The organic layer was washed with water, dried over $MgSO_4$, and evaporated at reduced pressure to give 0.32 g of (+)-3-(3,4-dichlorophenylthio)-2-(2-methoxycarbonylethyl)propionic acid as oil.

Specific rotation: $[\alpha]_D^{25}$ + 29.4° (c = 1.44, MeOH)

IR and NMR: The agreement with reference example 1.

In a further step, the above first filtrate was concentrated in vacuo and acidified with a 2N hydrochloric acid. The separated oil was extracted with ethyl acetate, washed with water, dried over $MgSO_4$, and ethyl acetate was evaporated at reduced pressure. The resulting residue (8.0 g), quinidine (7.7 g), and ethyl acetate (40 ml) was heated to solution, and about 20 ml of ethyl acetate was evaporated at reduced pressure. This residual solution was allowed to stand at room temperature. The precipitated crystals were collected, and further recrystallized from 2-propanol to give 11.7 g of the salt formed from (-)-3-(3,4-dichlorophenylthio)-2-(2-methoxycarbonylethyl)propionic acid and quinidine. To this salt (0.41 g) was added a 2N hydrochloric acid (15 ml), and a separated oil was extracted with ethyl acetate. The organic layer was washed with water, dried over $MgSO_4$, and evaporated at reduced pressure to give 0.20 g of (-)-3-(3,4-dichlorophenylthio)-2-(2-methoxycarbonylethyl)propionic acid as oil.

Specific rotation: $[\alpha]_D^{25}$ -28.3° (c = 1.02, MeOH)

IR and NMR spectrum were the agreement with reference example 1.

Reference example 4

3-(3,4-Dichlorophenylthio)-2-(3-methoxycarbonylpropyl)propionic acid

A mixture of 3,4-dichlorobenzenethiol (0.20 ml), di-tert-butyl 2-methyleneadipate (350 mg), ethanol (2 ml), and triton B (40% methanol solution, 2 drops) was stirred at 170°C in a sealed tube for 17 hours. After the reaction mixture was concentrated in vacuo, the resulting residue was dissolved in chloroform, washed with a 0.5% sodium hydroxide solution, water and a saturated sodium chloride solution respectively, and dried over $MgSO_4$. The solvent was evaporated at reduced pressure, and the residue was purified by flash column chromatography on silica eluting with diethyl ether/hexane (1:10) to give 430 mg of di-tert-butyl 2-(3,4-dichlorophenylthiomethyl)adipate as oil.

IR (neat): $\nu_{C=O}$ 1725 $cm^{-1}$

NMR ($CDCl_3$)

δ: 1.43(9H, s), 1.47(9H, s), 1.55-1.7(4H, m), 2.15-2.25(2H, m), 2.4-2.55(1H, m), 2.94(1H, dd, J = 6.0, 13.2Hz), 3.12(1H, dd, J = 8.2, 13.2Hz), 7.17(1H, dd, J = 2.2, 8.8Hz), 7.34(1H, d, J = 8.8Hz), 7.43(1H, d, J = 2.2Hz)

To a solution of di-tert-butyl 2-(3,4-dichlorophenylthiomethyl)adipate (550 mg) in benzene (7 ml) was added p-toluenesulfonic acid (40 mg), and the mixture was refluxed for 45 minutes. After the reaction mixture was cooled to room temperature, methanol (7 ml) was added, and the solution was stirred at 40°C for 2.5 hours. The reaction mixture was concentrated in vacuo, and resulting residue was purified by flash column chromatography on silica eluting with chloroform/methanol (10:1) to give 340 mg of 3-(3,4-dichlorophenylthio)-2-(3-methoxycarbonylpropyl)propionic acid as oil.

IR (neat): $\nu_{C=O}$ 1735, 1705 $cm^{-1}$

NMR ($CDCl_3$)

δ: 1.6-1.85(4H, m), 2.33(2H, t, J = 6.6Hz), 2.55-2.7(1H, m), 3.00(1H, dd, J = 6.0, 13.2Hz), 3.21(1H, dd, J = 7.7, 13.2Hz), 3.67(3H, s), 7.19(1H, dd, J = 2.2, 8.2Hz), 7.35(1H, d, J = 8.2Hz), 7.44(1H, d, J = 2.2Hz)

Reference example 5

3-(3,4-Dichlorophenylthio)-2-methoxycarbonylmethylpropionic acid

A mixture of 3,4-dichlorobenzenethiol (1.15 ml), sodium 3-methoxycarbonyl-2-methylenepropionate (1.50 g), methanol (40 ml), and triton B (40% methanol solution, 5 drops) was refluxed for 20 hours. The reaction mixture was concentrated in vacuo. To the resulting residue was added a diluted hydrochloric acid, and a separated oil was extracted with diethyl ether. The organic layer was washed with water, dried over $MgSO_4$, and evaporated at reduced pressure. The residue was purified by flash column chromatography on

silica eluting with chloroform/ethanol (10:1) to give 2.34 g of 3-(3,4-dichlorophenylthio)-2-methoxycarbonylmethylpropionic acid as oil.
IR (neat): $\nu_{C=O}$ 1740, 1710 $cm^{-1}$
NMR ($CDCl_3$)
δ: 2.65-2.9(2H, m), 3.0-3.2(2H, m), 3.3-3.45(1H, m), 3.69(3H, s), 7.20(1H, dd, J = 2.2, 8.2Hz), 7.36(1H, d, J = 8.2Hz), 7.46(1H, d, J = 2.2Hz)

Reference example 6

Methyl 5-(3,4-dichlorophenylthio)-4-(N,N-dipentylcarbamoyl)pentanoate

To a solution of 3-(3,4-dichlorophenylthio)-2-(2-methoxycarbonylethyl)propionic acid (2.90 g) in dry benzene (50 ml) was added thionyl chloride (1.0 ml), and the reaction mixture was refluxed for 2 hours. This solution was concentrated in vacuo. The residue was dissolved in dry dichloromethane (30 ml) and added dropwise into a solution of dipentylamine (1.8 ml) and triethylamine (1.8 ml) in dry dichloromethane (50 ml) with stirring at 0 ° C. After stirring at room temperature for 4 hours, the reaction mixture was washed with a dilute hydrochloric acid, a saturated sodium bicarbonate solution, and water respectively, dried over $MgSO_4$, and concentrated in vacuo. The residue was purified by flash column chromatography on silica eluting with chloroform to give 3.80 g of methyl 5-(3,4-dichlorophenylthio)-4-(N,N-dipentylcarbamoyl)-pentanoate as oil.
IR (neat): $\nu_{C=O}$ 1735, 1630 $cm^{-1}$
NMR ($CDCl_3$)
δ: 0.86(3H, t, 3 = 7.1Hz), 0.89(3H, t, J = 7.1Hz), 1.05-1.6(12H, m), 1.9-2.1(2H, m), 2.2-2.45(2H, m), 2.85-3.45-(7H, m), 3.67(3H, s), 7.16(1H, dd, J = 2.2, 8.2Hz), 7.34(1H, d, J = 8.2Hz), 7.40(1H, d, J = 2.2Hz)

Reference example 7

The compounds in the table were prepared in a similar manner to that described in reference example 6 (all compounds were oils). The compounds in which no specific rotation shows are in a racemate.

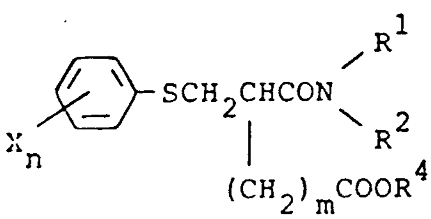

| $X_n$ | $R^1$ | $R^2$ | $R^4$ | m | Specific rotation $[\alpha]_D^{25}$ | IR ($cm^{-1}$) | NMR ($\delta$, $CDCl_3$) |
|---|---|---|---|---|---|---|---|
| 3,4-dichloro | pentyl | pentyl | methyl | 2 | +55.59° (C=1.18, MeOH) | the agreement with reference example 6 | |
| 3,4-dichloro | pentyl | pentyl | methyl | 2 | -57.64° (C=1.02, MeOH) | the agreement with reference example 6 | |
| 3,4-dichloro | 3-methoxy-propyl | pentyl | methyl | 2 | | 1730 1630 (C=O) | 0.86 and 0.89(3H, t, J=7.1Hz), 1.05-2.1(10H, m), 2.25-2.45 (2H, m), 2.85-3.5(12H, m), 3.67(3H, s), 7.16(1H, dd, J=2.2 8.8Hz), 7.34 and 7.35(1H, d, J=8.8Hz), 7.40(1H, d, J=2.2Hz) |
| 3,4-dichloro | 3-methoxy-propyl | pentyl | methyl | 2 | +54.26° (C=1.15, MeOH) | the same as mentioned above | |
| 3,4-dichloro | 3-methoxy-propyl | pentyl | methyl | 2 | -52.23° (C=1.03, MeOH) | the same as mentioned above | |

T-2

| $X_n$ | $R^1$ | $R^2$ | $R^4$ | m | Specific rotation $[\alpha]_D^{25}$ | IR ($cm^{-1}$) | NMR ($\delta$, $CDCl_3$) |
|---|---|---|---|---|---|---|---|
| 3,5-dichloro | pentyl | pentyl | methyl | 2 | | 1740 1640 (C=O) | 0.86(3H, t, J=7.2Hz), 0.89(3H, t, J=6.6Hz), 1.05-1.6(12H, m), 1.9-2.1(2H, m), 2.25-2.5(2H, m), 2.9-3.45(7H, m), 3.68(3H, s), 7.1-7.2(3H, m) |
| 4-chloro | pentyl | pentyl | methyl | 2 | | 1735 1635 (C=O) | 0.86(3H, t, J=7.1Hz), 0.89(3H, t, J=7.1Hz), 1.0-1.55(12H, m), 1.95-2.45(4H, m), 2.8-3.4(7H, m), 3.66(3H, s), 7.2-7.3(4H, m) |
| 3-chloro | pentyl | pentyl | methyl | 2 | | 1735 1640 (C=O) | 0.85(3H, t, J=7.1Hz), 0.89(3H, t, J=7.1Hz), 1.05-1.6(12H, m), 1.9-2.1(2H, m), 2.25-2.5(2H, m), 2.85-3.4(7H, m), 3.67(3H, s), 7.1-7.35(4H, m) |
| 3-bromo | pentyl | pentyl | methyl | 2 | | 1735 1640 (C=O) | 0.85(3H, t, J=7.1Hz), 0.89(3H, t, J=7.1Hz), 1.05-1.6(12H, m), 1.95-2.1(2H, m), 2.2-2.5(2H, m), 2.85-3.4(7H, m), 3.67(3H, s), 7.14(1H, t, J=7.7Hz), 7.2 7.35(2H, m), 7.45(1H, t, J=1.7Hz) |

T-3

| $X_n$ | $R^1$ | $R^2$ | $R^4$ | m | Specific rotation $[\alpha]_D^{25}$ | IR ($cm^{-1}$) | NMR (δ, $CDCl_3$) |
|---|---|---|---|---|---|---|---|
| 3,4-dichloro | pentyl | pentyl | methyl | 3 | | 1740 1640 (C=O) | 0.87(3H, t, J=7.1Hz), 0.89(3H, t, J=7.1Hz), 1.05-1.75(16H, m), 2.2-2.4(2H, m), 2.75-2.9 (1H, m), 2.95-3.4(6H, m), 3.67 (3H, s), 7.14(1H, dd, J=2.2, 8.2Hz), 7.34(1H, d, J=8.2Hz), 7.38(1H, d, J=2.2Hz) |
| 3,4-dichloro | pentyl | pentyl | methyl | 1 | | 1735 1640 (C=O) | 0.86(3H, t, J=7.1Hz), 0.88(3H, t, J=7.1Hz), 1.05-1.6(12H, m), 2.69(1H, dd, J=5.5, 16.5Hz), 2.78(1H, dd, J=6.6, 16.5Hz), 3.0-3.3(7H, m), 3.69(3H, s), 7.20 (1H, dd, J=2.2, 8.2Hz), 7.36(1H, d, J=8.2Hz), 7.46(1H, d, J=2.2Hz) |
| 3,4-dichloro | methyl | butyl | methyl | 2 | | 1740 1635 (C=O) | 0.85 and 0.93(3H, t, J=7.1Hz), 1.1-1.6(4H, m), 1.8-2.1(2H, m), 2.2-2.45(2H, m), 2.85-3.4(8H, m), 3.66(3H, s), 7.15 and 7.16(1H, dd, J=2.8, 8.2Hz), 7.34(1H, d, J=8.2Hz), 7.39 and 7.40(1H, d, J=2.8Hz) |

T-4

| $X_n$ | $R^1$ | $R^2$ | $R^4$ | m | Specific rotation $[\alpha]_D^{25}$ | IR ($cm^{-1}$) | NMR (δ, $CDCl_3$) |
|---|---|---|---|---|---|---|---|
| 3,4-dichloro | butyl | butyl | methyl | 2 | | 1735 1635 (C=O) | 0.84(3H, t, J=7.1Hz), 0.92(3H, t, J=7.1Hz), 1.1-1.55(8H, m), 1.9-2.1(2H, m), 2.2-2.5(2H, m), 2.85-3.45(7H, m), 3.67(3H, s), 7.16(1H, dd, J=2.2, 8.2Hz), 7.34(1H, d, J=8.2Hz), 7.40(1H, d, J=2.2Hz) |
| 3,4-dichloro | hexyl | hexyl | methyl | 2 | | 1735 1635 (C=O) | 0.87(6H, t, J=7.1Hz), 1.0-1.55 (16H, m), 1.9-2.1(2H, m), 2.2-2.5(2H, m), 2.85-3.45(7H, m), 3.67(3H, s), 7.15(1H, dd, J=2.2, 8.8Hz), 7.34(1H, d, J=8.8Hz), 7.39(1H, d, J=2.2Hz) |
| 3,4-dichloro | heptyl | heptyl | methyl | 2 | | 1730 1630 (C=O) | 0.8-0.95(6H, m), 1.05-1.55 (20H, m), 1.9-2.1(2H, m), 2.2-2.5(2H, m), 2.85-3.45(7H, m), 3.67(3H, s), 7.15(1H, dd, J=2.2, 8.8Hz), 7.34(1H, d, J=8.8Hz), 7.39(1H, d, J=2.2Hz) |

T-5

| $X_n$ | $R^1$ | $R^2$ | $R^4$ | m | Specific rotation $[\alpha]_D^{25}$ | IR ($cm^{-1}$) | NMR (δ, $CDCl_3$) |
|---|---|---|---|---|---|---|---|
| 3,4-dichloro | iso-pentyl | iso-pentyl | methyl | 2 | | 1740<br>1640<br>(C=O) | 0.79(3H, d, J=6.6Hz), 0.80(3H, d, J=6.6Hz), 0.92(6H, d, J=6.6Hz), 1.2-1.65(6H, m), 1.9-2.1(2H, m), 2.2-2.5(2H, m), 2.85-3.45(7H, m), 3.67(3H, s), 7.15(1H, dd, J=2.2, 8.2Hz), 7.34(1H, d, J=8.2Hz), 7.39(1H, d, J=2.2Hz) |
| 3,4-dichloro | pentyl | pentyl | ethyl | 2 | | 1730<br>1635<br>(C=O) | 0.86(3H, t, J=7.2Hz), 0.89(3H, t, J=6.6Hz), 1.05-1.6(15H, m), 1.9-2.1(2H, m), 2.2-2.45(2H, m), 2.85-3.45(7H, m), 4.13(2H, q, J=7.1Hz), 7.16(1H, dd, J=2.2, 8.2Hz), 7.34(1H, d, J=8.2Hz), 7.40(1H, d, J=2.2Hz) |

T-6

Reference example 8

Methyl 5-(3,4-dichlorophenylsulfinyl)-4-(N,N-dipentylcarbamoyl)pentanoate

Into a solution of methyl 5-(3,4-dichlorophenylthio)-4-(N,N-dipentylcarbamoyl)pentanoate (5.12 g) in dry dichloromethane (100 ml) was added by portions of m-chloroperbenzoic acid (70%, 2.65 g) with stirring at -78° C. After stirring at -78° C for 2 hours, sodium sulfite was added. The reaction mixture was washed with a saturated sodium bicarbonate solution and water, dried over $MgSO_4$, and concentrated in vacuo. The residue was purified by flash column chromatography on silica eluting with dichloromethane/diethyl ether/hexane (1:1:2) to give 2.39 g of methyl 5-(3,4-dichlorophenylsulfinyl)-4- (N,N-dipentylcarbamoyl)-pentanoate (diastereomer A) in a early fraction and 2.47 g of diastereomer B in a later fraction.

[Diastereomer A]

Melting point: 64-65° C

| Elemental Analysis (for $C_{23}H_{35}Cl_2NO_4S$): | | | |
|---|---|---|---|
| | C% | H% | N% |
| Calcd. | 56.09 | 7.16 | 2.84 |
| Found | 56.07 | 7.36 | 2.84 |

IR (KBr): $\nu_{C=O}$ 1730, 1630 $cm^{-1}$
$\nu_{SO}$ 1040 $cm^{-1}$
NMR ($CDCl_3$)
δ: 0.90 (3H, t, J=7.1Hz), 0.94(3H, t, J=7.1Hz), 1.2-2.1(14H, m), 2.33(2H, t, J=7.1Hz), 2.74(1H, dd, J=2.8, 12.1Hz), 3.2-3.6(6H, m), 3.66(3H, s), 7.47(1H, dd, J=2.2, 8.2Hz), 7.60(1H, d, J=8.2Hz), 7.78(1H, d, J=2.2Hz)

[Diastereomer B]

Property: oil
IR (neat) : $\nu_{C=O}$ 1735, 1635 $cm^{-1}$
$\nu_{SO}$ 1050 $cm^{-1}$
NMR ($CDCl_3$)
δ: 0.87(3H, t, J=7.1Hz), 0.94(3H, t, J=7.1Hz), 1.15-1.65(12H, m), 2.05-2.6(4H, m), 2.85-3.4(7H, m), 3.69(3H, s), 7.39(1H, dd, J=2.2, 8.2Hz), 7.58(1H, d, J=8.2Hz), 7.74(1H, d, J=2.2Hz)

Reference example 9

The compounds in the table were prepared in a similar manner to that described in reference example 8.

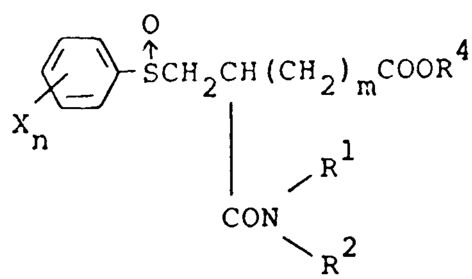

| $X_n$ | $R^1$ | $R^2$ | $R^4$ | m | diastereomer (note) | mp (°C) (recryst. solvent) | IR ($cm^{-1}$) | NMR (δ, $CDCl_3$) |
|---|---|---|---|---|---|---|---|---|
| 3,5-dichloro | pentyl | pentyl | methyl | 2 | A | oil | 1740<br>1635<br>(C=O)<br>1055<br>(S-O) | 0.85-1.0(6H, m), 1.2-2.1 (14H, m), 2.33(2H, t, J=7.2Hz), 2.75(1H, dd, J=2.7, 12.1Hz), 3.2-3.6(6H, m), 3.67 (3H, s), 7.46(1H, t, J=2.2Hz), 7.53(2H, d, J=2.2Hz) |
| 3,5-dichloro | pentyl | pentyl | methyl | 2 | B | oil | 1735<br>1630<br>(C=O)<br>1050<br>(S-O) | 0.88(3H, t, J=7.1Hz), 0.94(3H, t, J=7.1Hz), 1.15-1.7(12H, m), 2.1-2.5(4H, m), 2.92(1H, dd, J=8.2, 13.7Hz), 3.05-3.4 (6H, m), 3.69(3H, s), 7.44(1H, t, J=1.7Hz), 7.48(2H, d, J=1.7Hz) |
| 4-chloro | pentyl | pentyl | methyl | 2 | A | oil | 1740<br>1635<br>(C=O)<br>1045<br>(S-O) | 0.90(3H, t, J=7.1Hz), 0.93(3H, t, J=7.1Hz), 1.2-2.1(14H, m), 2.32(2H, t, J=7.1Hz), 2.74(1H, dd, J=3.3, 12.1Hz), 3.15-3.55 (6H, m), 3.66(3H, s), 7.45-7.65(4H, m) |

(note) Diastereomer A is a product isolated by flash column chromatography on silica in a early fraction and diastereomer B is a product in a later fraction.

T-7

| $X_n$ | $R^1$ | $R^2$ | $R^4$ | m | diastereomer (note) | mp (°C) (recryst. solvent) | IR ($cm^{-1}$) | NMR (δ, $CDCl_3$) |
|---|---|---|---|---|---|---|---|---|
| 4-chloro | pentyl | pentyl | methyl | 2 | B | oil | 1735 1635 (C=O) 1040 (S-O) | 0.87(3H, t, J=7.1Hz), 0.93(3H, t, J=7.1Hz), 1.1-1.65(12H, m), 2.1-2.5(4H, m), 2.87(1H, dd, J=8.8, 13.7Hz), 3.02(1H, dd, J=4.4, 13.7Hz), 3.1-3.4 (5H, m), 3.69(3H, s), 7.45-7.6(4H, m) |
| 3-chloro | pentyl | pentyl | methyl | 2 | A | oil | 1735 1640 (C=O) 1045 (S-O) | 0.90(3H, t, J=7.1Hz), 0.94(3H, t, J=6.6Hz), 1.2-2.1(14H, m), 2.32(2H, t, J=7.1Hz), 2.75(1H, dd, J=2.8, 12.1Hz), 3.2-3.55 (6H, m), 3.66(3H, s), 7.4-7.55(3H, m), 7.68(1H, s) |
| 3-chloro | pentyl | pentyl | methyl | 2 | B | oil | 1735 1630 (C=O) 1045 (S-O) | 0.87(3H, t, J=7.1Hz), 0.94(3H, t, J=7.1Hz), 1.15-1.65(12H, m), 2.15 and 2.17(2H, t, J=7.1Hz), 2.25-2.5(2H, m), 2.89 (1H, dd, J=8.8, 13.7Hz), 3.05 (1H, dd, J=4.4, 13.7Hz), 3.1-3.4(5H, m), 3.69(3H, s), 7.4-7.55(3H, m), 7.65(1H, s) |

(note) Diastereomer A is a product isolated by flash column chromatography on silica in a early fraction and diastereomer B is a product in a later fraction.

T - 8

| $X_n$ | $R^1$ | $R^2$ | $R^4$ | m | diastereomer (note) | mp (°C) (recryst. solvent) | IR ($cm^{-1}$) | NMR (δ, $CDCl_3$) |
|---|---|---|---|---|---|---|---|---|
| 3-bromo | pentyl | pentyl | methyl | 2 | A | oil | 1730 1630 (C=O) 1040 (S-O) | 0.90(3H, t, J=7.1Hz), 0.94(3H, t, J=7.1Hz), 1.2-2.1(14H, m), 2.33(2H, t, J=7.1Hz), 2.75(1H, dd, J=2.8, 12.1Hz), 3.2-3.55 (6H, m), 3.66(3H, s), 7.38(1H, t, J=7.7Hz), 7.5-7.65(2H, m), 7.82(1H, t, J=1.7Hz) |
| 3-bromo | pentyl | pentyl | methyl | 2 | B | oil | 1735 1635 (C=O) 1045 (S-O) | 0.87(3H, t, J=7.1Hz), 0.94(3H, t, J=7.1Hz), 1.15-1.7(12H, m), 2.15 and 2.17(2H, t, J=7.1Hz), 2.25-2.5(2H, m), 2.89 (1H, dd, J=8.8, 13.7Hz), 3.05 (1H, dd, J=4.4, 13.7Hz), 3.1-3.4(5H, m), 3.69(3H, s), 7.37 (1H, t, J=7.7Hz), 7.45-7.65 (2H, m), 7.79(1H, t, J=1.7Hz) |
| 3,4-dichloro | pentyl | pentyl | methyl | 1 | A | oil | 1735 1640 (C=O) 1050 (S-O) | 0.8-1.0(6H, m), 1.05-1.8 (12H, m), 2.49(1H, dd, J=7.1, 15.9Hz), 2.74(1H, dd, J=6.6, 15.9Hz), 2.88(1H, dd, J=4.4, 12.6Hz), 3.1-3.45(5H, m), 3.6-3.75(4H, m), 7.44(1H, dd, J=2.2, 8.2Hz), 7.60(1H, d, J=8.2Hz), 7.76(1H, d, J=2.2Hz) |

(note) Diastereomer A is a product isolated by flash column chromatography on silica in a early fraction and diastereomer B is a product in a later fraction.

T-9

| $X_n$ | $R^1$ | $R^2$ | $R^4$ | m | diastereomer (note) | mp (°C) (recryst. solvent) | IR ($cm^{-1}$) | NMR (δ, $CDCl_3$) |
|---|---|---|---|---|---|---|---|---|
| 3,4-dichloro | pentyl | pentyl | methyl | 1 | B | 65-68 (hexane) | 1730 1635 (C=O) 1050 (S-O) | 0.86(3H, t, J=7.1Hz), 0.94(3H, t, J=7.1Hz), 1.1-1.75(12H, m), 2.8-3.05(4H, m), 3.1-3.35(4H, m), 3.6-3.8(4H, m), 7.40(1H, dd, J=2.2, 8.2Hz), 7.60(1H, d, J=8.2Hz), 7.74(1H, d, J=2.2Hz) |
| 3,4-dichloro | pentyl | pentyl | methyl | 3 | A | oil | 1740 1630 (C=O) 1050 (S-O) | 0.90(3H, t, J=7.1Hz), 0.94(3H, t, J=7.1Hz), 1.2-1.8(16H, m), 2.2-2.35(2H, m), 2.65-2.8 (1H, m), 3.2-3.55(6H, m), 3.65(3H, s), 7.45(1H, dd, J=2.2, 8.2Hz), 7.60(1H, d, J=8.2Hz), 7.76(1H, d, J=2.2Hz) |
| 3,4-dichloro | pentyl | pentyl | methyl | 3 | B | oil | 1740 1635 (C=O) 1045 (S-O) | 0.87(3H, t, J=7.1Hz), 0.94(3H, t, J=7.1Hz), 1.15-1.90(16H, m), 2.35(2H, t, J=7.1Hz), 2.93 (1H, dd, J=8.2, 13.7Hz), 3.05-3.35(6H, m), 3.68(3H, s), 7.37(1H, dd, J=2.2, 8.2Hz), 7.57(1H, d, J=8.2Hz), 7.73(1H, d, J=2.2Hz) |

(note) Diastereomer A is a product isolated by flash column chromatography on silica in a early fraction and diastereomer B is a product in a later fraction.

T -10

| $X_n$ | $R^1$ | $R^2$ | $R^4$ | m | diastereomer (note) | mp (°C) (recryst. solvent) | IR ($cm^{-1}$) | NMR (δ, $CDCl_3$) |
|---|---|---|---|---|---|---|---|---|
| 3,4-dichloro | butyl | butyl | methyl | 2 | A | oil | 1740<br>1640<br>(C=O)<br>1050<br>(S-O) | 0.85-1.05(6H, m), 1.2-2.15 (10H, m), 2.33(2H, t, J=7.1Hz), 2.74(1H, dd, J=2.7, 12.1Hz), 3.2-3.6(6H, m), 3.67 (3H, s), 7.46(1H, dd, J=2.2, 8.2Hz), 7.60(1H, d, J=8.2Hz), 7.77(1H, d, J=2.2Hz) |
| 3,4-dichloro | butyl | butyl | methyl | 2 | B | oil | 1735<br>1630<br>(C=O)<br>1050<br>(S-O) | 0.90(3H, t, J=7.1Hz), 0.97(3H, t, J=7.1Hz), 1.15-1.7(8H, m), 2.05-2.5(4H, m), 2.91(1H, dd, J=7.7, 13.7Hz), 3.0-3.4 (6H, m), 3.69(3H, s), 7.38(1H, dd, J=2.2, 8.2Hz), 7.58(1H, d, J=8.2Hz), 7.74(1H, d, J=2.2Hz) |
| 3,4-dichloro | hexyl | hexyl | methyl | 2 | A | oil | 1740<br>1640<br>(C=O)<br>1050<br>(S-O) | 0.8-1.0(6H, m), 1.2-2.1 (18H, m), 2.32(2H, t, J=7.1Hz), 2.74(1H, dd, J=2.8, 12.1Hz), 3.2-3.6(6H, m), 3.66 (3H, s), 7.46(1H, dd, J=2.2, 8.2Hz), 7.60(1H, d, J=8.2Hz), 7.77(1H, d, J=2.2Hz) |

(note) Diastereomer A is a product isolated by flash column chromatography on silica in a early fraction and diastereomer B is a product in a later fraction.

T-11

| $X_n$ | $R^1$ | $R^2$ | $R^4$ | m | diastereomer (note) | mp (°C) (recryst. solvent) | IR ($cm^{-1}$) | NMR (δ, $CDCl_3$) |
|---|---|---|---|---|---|---|---|---|
| 3,4-dichloro | hexyl | hexyl | methyl | 2 | B | 77-78 (diethyl ether-hexane) | 1725 1625 (C=O) 1060 (S-O) | 0.8-1.0(6H, m), 1.15-1.7 (16H, m), 2.05-2.5(4H, m), 2.91(1H, dd, J=8.2, 13.7Hz), 3.0-3.4(6H, m), 3.69(3H, s), 7.38(1H, dd, J=2.2, 8.2Hz), 7.57(1H, d, J=8.2Hz), 7.73(1H, d, J=2.2Hz) |
| 3,4-dichloro | pentyl | pentyl | ethyl | 2 | A | oil | 1730 1630 (C=O) 1050 (S-O) | 0.90(3H, t, J=7.1Hz), 0.94(3H, t, J=6.6Hz), 1.15-2.1(17H, m), 2.31(2H, t, J=7.1Hz), 2.75 (1H, dd, J=3.3, 12.1Hz), 3.15-3.6(6H, m), 4.12(2H, q, J=7.1Hz), 7.47(1H, dd, J=2.2, 8.2 Hz), 7.60(1H, d, J=8.2Hz), 7.78(1H, d, J=2.2Hz) |

(note) Diastereomer A is a product isolated by flash column chromatography on silica in a early fraction and diastereomer B is a product in a later fraction.

T-12

Reference example 10

Methyl 5-(3,4-dichlorophenylthio)-4-(N-pentyl-N-phenethylcarbamoyl)pentanoate

To a solution of 3-(3,4-dichlorophenylthio)-2-(2-methoxycarbonylethyl)propionic acid (190 mg) in dry benzene (3 ml) was added thionyl chloride (0.1 ml), and the mixture was refluxed for 2 hours. The reaction mixture was concentrated in vacuo, and the residue which was dissolved in dry dichloromethane (3 ml) was added dropwise into a solution of N-pentylphenethylamine (110 mg) and triethylamine (0.1 ml) in dry dichloromethane (3 ml) with stirring at 0 ° C. After stirring at room temperature for 4 hours, the reaction mixture was washed with a dilute hydrochloric acid, water, a saturated sodium bicarbonate solution, and water respectively, dried over $MgSO_4$, and concentrated in vacuo. The residue was purified by flash column chromatography on silica eluting with dichloromethane/diethyl ether/hexane (1:1:4) to give 280 mg of methyl 5-(3,4-dichlorophenylthio)-4-(N-penyl-N-phenethylcarbamoyl)pentanoate as oil.

IR (neat): $\nu_{C=O}$ 1730, 1635 $cm^{-1}$
NMR ($CDCl_3$)
δ: 0.84 and 0.89(3H, t, J=6,6Hz), 1.0-1.6(6H, m), 1.85-2.1(2H, m), 2.2-2.45(2H, m), 2.65-3.7(12H, m), 6.97-(1H, d, J=6.6Hz), 7.1-7.4(7H, m)

Reference example 11

The compounds in the table were prepared in a similar manner to that described in reference example 10 (all compounds were oils)

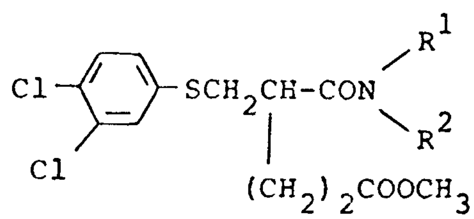

| $R^1$ | $R^2$ | IR ($cm^{-1}$) | NMR (δ, $CDCl_3$) |
|---|---|---|---|
| pentyl | 3-phenylpropyl | 1730, 1630 (C=O) | 0.85 and 0.87(3H, t, J=6.6Hz), 1.0-1.55(6H, m), 1.7-2.1(4H, m), 2.2-2.65(4H, m), 2.8-3.45(7H, m), 3.63 and 3.66(3H, s), 7.05-7.4(8H, m) |
| phenethyl | phenethyl | 1730, 1635 (C=O) | 1.85-2.05(2H, m), 2.15-2.4(2H, m), 2.55-3.7(14H, m), 6.93 (2H, d, J=6.6Hz), 7.12(1H, dd, J=2.2, 8.8Hz), 7.15-7.35(9H, m), 7.38(1H, d, J=2.2Hz) |
| 3-phenylpropyl | 3-phenylpropyl | 1735, 1635 (C=O) | 1.7-2.05(6H, m), 2.15-2.4(2H, m), 2.46(2H, t, J=7.7Hz), 2.59(2H, t, J=7.7Hz), 2.8-3.45(7H, m), 3.62(3H, s), 7.0-7.4(13H, m) |
| hexyl | 4-phenylbutyl | 1735, 1635 (C=O) | 0.87(3H, t, J=7.2Hz), 1.0-1.7(12H, m), 1.85-2.1(2H, m), 2.15-2.7(4H, m), 2.85-3.45(7H, m), 3.65 and 3.66(3H, s), 7.1-7.4(8H, m) |
| methyl | benzyl | 1730, 1670, 1635 (C=O) | 1.9-2.5(4H, m), 2.8-3.35(6H, m), 3.60 and 3.65(3H, s), 4.35-4.75(2H, m), 7.0-7.4(8H, m) |

T-13

Reference example 12

Methyl 5-(3,4-dichlorophenylsulfinyl)-4-(N-pentyl-N-phenethylcarbamoyl)pentanoate

Into a solution of methyl 5-(3,4-dichlorophenylthio)-4-(N-pentyl-N-phenethylcarbamoyl)pentanoate (280

mg) in dry chloroform (10 ml) was added by portions of m-chloroperbenzoic acid (70%, 140 mg) with stirring at -78° C. After stirring at -78° C for 2 hours, sodium sulfite was added. The reaction mixture was washed with a saturated sodium bicarbonate solution and water, dried over $MgSO_4$, and concentrated in vacuo. The residue was purified by flash column chromatography on silica eluting with dichloromethane/diethy ether/hexane (1:1:2) to give 100 mg of methyl 5-(3,4-dichlorophenylsulfinyl)-4-(N-pentyl-N-phenethylcarbamoyl)pentanoate (diastereomer A) in a early fraction and 110 mg of diastereomer B in a later fraction.

[Diastereomer A]

Property: oil
IR (neat): $\nu_{C=O}$ 1735, 1630 $cm^{-1}$
$\nu_{SO}$ 1050 $cm^{-1}$
NMR ($CDCl_3$)
δ: 0.90 and 0.92(3H, t, J=7.1Hz), 1.2-2.05(8H, m), 2.28(2H, t, J=7.1Hz), 2.7-3.05(3H, m), 3.15-3.85(9H, m), 7.15-7.4(5H, m), 7.46(1H, dd, J=1.7, 8.2Hz), 7.60(1H, d, J=8.2Hz), 7.77(1H, d, J=1.7Hz)

[Diastereomer B]

Property: oil
IR (neat): $\nu_{C=O}$ 1740, 1630 $cm^{-1}$
$\nu_{SO}$ 1045 $cm^{-1}$
NMR ($CDCl_3$)
δ: 0.87 and 0.92(3H, t, J=7.1Hz), 1.15-1.65(8H, m), 2.05-2.45(4H, m), 2.7-3.0(3H, m), 3.1-3.6(4H, m), 3.66 and 3.69(3H, s), 7.1-7.45(6H, m), 7.57 and 7.59(1H, d, J=8.2Hz), 7.73(1H, d, J=1.6Hz)

Reference example 13

The compounds in the table were prepared in a similar manner to that described in reference example 12 (all compounds were oils).

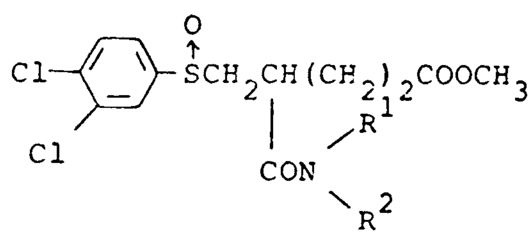

| $R^1$ | $R^2$ | diastereomer (note) | IR ($cm^{-1}$) | NMR (δ, $CDCl_3$) |
|---|---|---|---|---|
| pentyl | 3-phenylpropyl | A | 1735, 1630 (C=O) 1045 (S-O) | 0.88 and 0.92(3H, t, J=6.6Hz), 1.15-2.4(12H, m), 2.55-2.8(3H, m), 3.15-3.7(9H, m), 7.15-7.4(5H, m), 7.45 and 7.46(1H, dd, J=1.7, 8.2Hz), 7.60(1H, d, J=8.2Hz), 7.76 and 7.77(1H, d, J=1.7Hz) |
| pentyl | 3-phenylpropyl | B | 1730, 1630 (C=O) 1045 (S-O) | 0.85 and 0.92(3H, t, J=7.1Hz), 1.1-2.5(12H, m), 2.56 and 2.65(2H, t, J=7.7Hz), 2.89(1H, dd, J=8.2, 13.7Hz), 3.0-3.35(6H, m), 3.66 and 3.68(3H, s), 7.1-7.4(6H, m), 7.55 and 7.56(1H, d, J=8.2Hz), 7.73 (1H, d, J=2.2Hz) |
| phenethyl | phenethyl | A | 1730, 1635 (C=O) 1050 (S-O) | 1.7-2.0(2H, m), 2.23(2H, t, J=7.1Hz), 2.74(1H, dd, J=3.3, 12.1Hz), 2.8-3.0(4H, m), 3.15-3.9(9H, m), 7.15-7.4(10H, m), 7.46(1H, dd, J=2.2, 8.2Hz), 7.60 (1H, d, J=8.2Hz), 7.77(1H, d, J=2.2Hz) |
| phenethyl | phenethyl | B | 1735, 1635 (C=O) 1050 (S-O) | 2.0-2.35(4H, m), 2.65-2.85(6H, m), 3.15-3.7(8H, m), 7.1-7.75(13H, m) |
| 3-phenylpropyl | 3-phenylpropyl | A | 1740, 1640 (C=O) 1055 (S-O) | 1.7-2.35(8H, m), 2.55-2.75(5H, m), 3.2-3.6(6H, m), 3.62(3H, s), 7.15-7.35(10H, m), 7.45(1H, dd, J=2.2, 8.2Hz), 7.59(1H, d, J=8.2Hz), 7.77(1H, d, J=2.2Hz) |

(note) Diastereomer A is a product isolated by flash column chromatography on silica in a early fraction and diastereomer B is a product in a later fraction.

T-14

| $R^1$ | $R^2$ | diastereomer (note) | IR ($cm^{-1}$) | NMR (δ, $CDCl_3$) |
|---|---|---|---|---|
| 3-phenylpropyl | 3-phenylpropyl | B | 1730, 1635 (C=O) 1045 (S-O) | 1.6-2.15(6H, m), 2.2-2.45(2H, m), 2.53(2H, t, J=7.7Hz), 2.62(2H, t, J=7.7Hz), 2.87(1H, dd, J=7.7, 13.7Hz), 3.05-3.35(6H, m), 3.65(3H, s), 7.1-7.4 (11H, m), 7.54(1H, d, J=8.2Hz), 7.72(1H, d, J=2.2Hz) |

(note) Diastereomer A is a product isolated by flash column chromatography on silica in a early fraction and diastereomer B is a product in a later fraction.

T-15

Reference example 14

3-(3,4-Dimethylphenylthio)-2-(2-methoxycarbonylethyl)propionic acid

A mixture of 3,4-dimethylbenzenethiol (0.59 ml), 2-methyleneglutaronitrile (0.48 ml), ethanol (10 ml), and triton B (40% ethanol solution, 5 drops) was refluxed for 5 hours. After the reaction mixture was concentrated in vacuo, the resulting residue was dissolved in chloroform, washed with water, and dried over $MgSO_4$. The solvent was evaporated at reduced pressure, and the residue was purified by flash column chromatography on silica eluting with benzene/ethyl acetate (7:1) to give 1.18 g of 2-(3,4-dimethylphenylthiomethyl)glutaronitrile as oil.

IR (neat): $\nu_{CN}$ 2230 $cm^{-1}$

NMR ($CDCl_3$)

δ: 1.9-2.35(8H, m), 2.45-2.7(2H, m), 2.85-2.9(1H, m), 2.98(1H, dd, J=7.7, 13.7Hz), 3.18(1H, dd, J=6.6, 13.7Hz), 7.11(1H, d, J=7.7Hz), 7.19(1H, dd, J=1.7, 7.7Hz), 7.24(1H, d, J=1.7Hz)

To a solution of 2-(3,4-dimethylphenylthiomethyl)-glutaronitrile (1.16 g) in acetic acid (8 ml) was added a concentrated hydrochloric acid (8 ml), and the mixture was refluxed for 19 hours. After the reaction mixture was concentrated in vacuo, diethyl ether was added, and the precipitates formed were filtered off. The filtrate was washed with water, and shaken with a saturated sodium bicarbonate solution. The aqueous layer was acidified with a concentrated hydrochloric acid, and extracted with diethyl ether. The organic layer was washed with water, dried over $MgSO_4$, and evaporated at reduced pressure. The residue was recrystallized from diethyl ether-hexane to give 1.18 g of 2-(3,4-dimethylphenylthiomethyl)glutaric acid.

Melting point: 96-98 °C

| Elemental Analysis (for $C_{14}H_{18}O_4S$): | | |
|---|---|---|
| | C% | H% |
| Calcd. | 59.55 | 6.43 |
| Found | 59.38 | 6.56 |

IR (KBr): $\nu_{C=O}$ 1705 $cm^{-1}$

NMR (DMSO-$d_6$)

δ: 1.65-1.95(2H, m), 2.1-2.55(9H, m), 3.04(2H, d, J=7.2Hz), 7.0-7.2(3H, m), 12.28(2H, s)

A mixture of 2-(3,4-dimethylphenylthiomethyl)-glutaric acid (1.16 g), methanol (20 ml), and p-toluenesulfonic acid (0.04 g) was stirred at 40 °C for 1.5 hours. The reaction mixture was concentrated in vacuo, and the residue was purified by flash column chromatography on silica eluting with chloroform/ethanol (10:1) to give 0.98 g of 3-(3,4-dimethylphenylthio)-2-(2-methoxycarbonylethyl)propionic acid as oil.

IR (neat): $\nu_{C=O}$ 1735, 1705 $cm^{-1}$

NMR ($CDCl_3$)

δ: 1.9-2.5(10H, m), 2.55-2.7(1H, m), 2.94(1H, dd, J=6.6, 13.7Hz), 3.19(1H, dd, J=7.7, 13.7Hz), 3.65(3H, s), 7.06(1H, d, J=7.7Hz), 7.1-7.2(2H, m)

Reference example 15

The compounds in the table were prepared in a similar manner to that discribed in reference example 14.

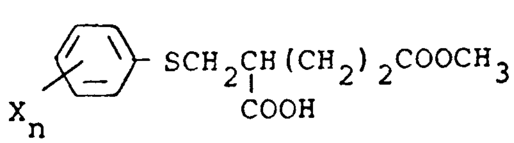

| $X_n$ | mp (°C) (recryst. solvent) | IR ($cm^{-1}$) | NMR (δ, $CDCl_3$) |
|---|---|---|---|
| 4-methyl | oil | 1735, 1710 (C=O) | 1.95-2.15(2H, m), 2.25-2.5(5H, m), 2.55-2.7(1H, m), 2.95(1H, dd, J=6.6, 13.2Hz), 3.19(1H, dd, J=7.7, 13.2Hz), 3.65(3H, s), 7.11(2H, d, J=7.7Hz), 7.27(2H, d, J=7.7Hz) |
| 4-nitro | oil | 1735, 1710 (C=O) 1510 ($NO_2$) | 2.0-2.2(2H, m), 2.35-2.55(2H, m), 2.7-2.85(1H, m), 3.16(1H, dd, J=6.6, 13.2Hz), 3.36(1H, dd, J=7.7, 13.2Hz), 3.68(3H, s), 7.35-7.45(2H, m), 8.1-8.2(2H, m) |
| 4-acetyl | 59.5-61.0 (dichloromethane-hexane) | 1725, 1690, 1680 (C=O) | 1.95-2.15(2H, m), 2.35-2.5(2H, m), 2.57(3H, s), 2.7-2.85(1H, m), 3.11(1H, dd, J=6.6, 13.7Hz), 3.33(1H, dd, J=7.7, 13.7Hz), 3.67(3H, s), 7.37(2H, d, J=8.8Hz), 7.88(2H, d, J=8.8Hz) |

T-15

Reference example 16

Methyl 5-(3,4-dimethylphenylthio)-4-(N,N-dipentylcarbamoyl)pentanoate

To a solution of 3-(3,4-dimethylphenylthio)-2-(2-methoxycarbonylethyl)propionic acid (0.50 g) in dry benzene (6 ml) was added thionyl chloride (0.2 ml), and the reaction mixture was refluxed for 2 hours. This solution was concentrated in vacuo. The residue was dissolved in dry dichloromethane (4 ml) and added dropwise into a solution of dipentylamine (0.35 ml) and triethylamine (0.3 ml) in dry dichloromethane (2 ml) with stirring at 0 ° C. After stirring at room temperature for 5 hours, the reaction mixture was washed with a dilute hydrochloric acid, water, a saturated sodium bicarbonate solution, and water respectively, dried over $MgSO_4$, and concentrated in vacuo. The residue was purified by flash column chromatography on silica eluting with chloroform to give 0.76 g of methyl 5-(3,4-dimethylphenylthio)-4-(N,N-dipentylcarbamoyl)-pentanoate as oil.

IR (neat): $\nu_{C=O}$ 1730, 1635 $cm^{-1}$

NMR ($CDCl_3$)

δ: 0.84(3H, t, J = 6.6Hz), 0.89(3H, t, J = 6.6Hz), 0.95-1.6(12H, m), 1.95-2.1(2H, m), 2.15-2.5(8H, m), 2.8-3.4-(7H, m), 3.65(3H, s), 7.0-7.2(3H, m)

Reference example 17

The compunds in the table were prepared in a similar manner to that described in reference example 16 (all compounds were oils).

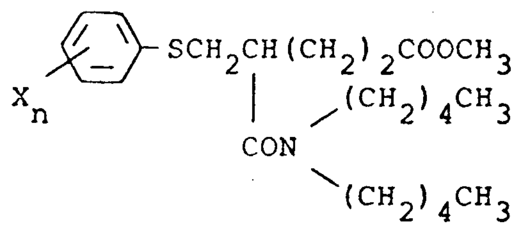

| $X_n$ | IR ($cm^{-1}$) | NMR (δ, $CDCl_3$) |
|---|---|---|
| 4-methyl | 1730, 1635 (C=O) | 0.84(3H, t, J=7.1Hz), 0.89(3H, t, J=7.1Hz), 0.95-1.6(12H, m), 1.95-2.1(2H, m), 2.15-2.45(5H, m), 2.75-3.4(7H, m), 3.65(3H, s), 7.10(2H, d, J=8.2Hz), 7.27(2H, d, J=8.2Hz) |
| 4-nitro | 1735, 1635 (C=O) 1515 ($NO_2$) | 0.81(3H, t, J=6.6Hz), 0.88(3H, t, J=6.6Hz), 1.0-1.6(12H, m), 1.9-2.15(2H, m), 2.25-2.5(2H, m), 3.0-3.45(7H, m), 3.69(3H, s), 7.35(2H, d, J=8.8Hz), 8.13(2H, d, J=8.8Hz) |
| 4-acetyl | 1735, 1685, 1635 (C=O) | 0.80(3H, t, J=6.6Hz), 0.89(3H, t, J=6.6Hz), 1.0-1.6(12H, m), 1.9-2.1(2H, m), 2.25-2.5(2H, m), 2.56(3H, s), 2.9-3.45(7H, m), 3.67(3H, s), 7.32(2H, d, J=8.2Hz), 7.86(2H, d, J=8.2Hz) |

T- 17

Reference example 18

Methyl 5-(3,4-dimethylphenylsufinyl)-4-(N,N-dipentylcarbamoyl)pentanoate

Into a solution of methyl 5-(3,4-dimethylphenylthio)-4-(N,N-dipentylcarbamoyl)pentanoate (0.55 g) in dry dichloromethane (10 ml) was added by portions of m-chloroperbenzoic acid (70%, 0.31 g) with stirring at -78°C. After stirring at -78°C for 3 hours, sodium sulfite was added. The reaction mixture was washed with a saturated sodium bicarbonate solution and water, dried over $MgSO_4$, and concentrated in vacuo. The residue was purified by flash column chromatography on silica eluting with benzene/ethyl acetate (2:1) to give 0.12 g of methyl 5-(3,4-dimethylphenylsulfinyl)-4-(N,N-dipentylcarbamoyl)pentanoate (diastereomer A) in a early fraction and 0.36 g of diastereomer B in a later fraction.

[Diastereomer A]

Property: oil
IR (neat): $\nu_{C=O}$ 1730, 1630 $cm^{-1}$
$\nu_{SO}$ 1045 $cm^{-1}$
NMR ($CDCl_3$)
δ: 0.90(3H, t, J=6.6Hz), 0.93(3H, t, J=6.6Hz), 1.1-2.45(22H, m), 2.76(1H, dd, J=3.3, 12.1Hz), 3.1-3.5(6H, m), 3.65(3H, s), 7.2-7.45(3H, m)

[Diastereomer B]

Property: oil
IR (neat): $\nu_{C=O}$ 1735, 1635 $cm^{-1}$
$\nu_{SO}$ 1045 $cm^{-1}$
NMR ($CDCl_3$)
δ: 0.87(3H, t, J=7.1Hz), 0.93(3H, t, J=7.1Hz), 1.15-1.65(12H, m), 2.1-2.5(10H, m), 2.82(1H, dd, J=9.3, 13.2Hz), 2.97(1H, dd, J=3.8, 13.2Hz), 3.1-3.45(5H, m), 3.68(3H, s), 7.2-7.45(3H, m)

Reference example 19

The compounds in the table were prepared in a similar manner to that discribed reference example 18 (all compounds were oils)

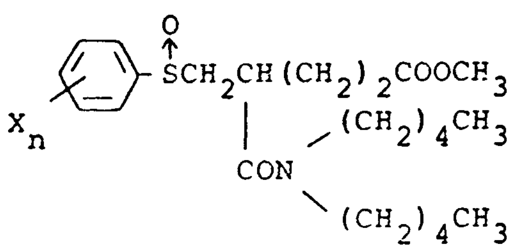

| $X_n$ | diastereomer (note) | IR ($cm^{-1}$) | NMR (δ, $CDCl_3$) |
|---|---|---|---|
| 4-methyl | A | 1740, 1635 (C=O) 1045 (S-O) | 0.90(3H, t, J=7.1Hz), 0.93(3H, t, J=7.1Hz), 1.15-2.1(14H, m), 2.25-2.45(5H, m), 2.76(1H, dd, J=3.3, 12.1Hz), 3.1-3.5(6H, m), 3.65(3H, s), 7.32(2H, d, J=8.2Hz), 7.53(2H, d, J=8.2Hz) |
| 4-methyl | B | 1730, 1635 (C=O) 1040 (S-O) | 0.87(3H, t, J=7.1Hz), 0.93(3H, t, J=7.1Hz), 1.1-1.7(12H, m), 2.1-2.5(7H, m), 2.83(1H, dd, J=9.3, 13.7Hz), 2.98(1H, dd, J=3.8, 13.7Hz), 3.1-3.45(5H, m), 3.68(3H, s), 7.31(2H, d, J=8.2Hz), 7.49(2H, d, J=8.2Hz) |
| 4-nitro | A | 1735, 1635 (C=O) 1525 ($NO_2$) 1045 (S-O) | 0.91(3H, t, J=7.1Hz), 0.94(3H, t, J=7.1Hz), 1.2-2.1(14H, m), 2.33 (2H, t, J=7.2Hz), 2.77(1H, dd, J=2.8, 12.1Hz), 3.2-3.6(6H, m), 3.66 (3H, s), 7.86(2H, d, J=8.8Hz), 8.38(2H, d, J=8.8Hz) |
| 4-nitro | B | 1730, 1630 (C=O) 1525 ($NO_2$) 1050 (S-O) | 0.86(3H, t, J=7.1Hz), 0.94(3H, t, J=7.1Hz), 1.1-1.7(12H, m), 2.1-2.55(4H, m), 2.9-3.45(7H, m), 3.70(3H, s), 7.80(2H, d, J=8.8Hz), 8.36 (2H, d, J=8.8Hz) |

(note) Diastereomer A is a product isolated by flash column chromatography on silica in a early fraction and diastereomer B is a product in a later fraction.

T-18

Example 1

Methyl 5-(3,4-dichlorophenylsulfonyl)-4-(N,N-dipentylcarbamoyl)pentanoate

Into a solution of methyl 5-(3,4-dichlorophenylthin)-4-(N,N-dipentylcarbamoyl)pentanoate (54.3 g) in dry

dichloromethane (500 ml) was added by portions of m-chloroperbenzoic acid (80%, 59.5 g) with stirring at 0°C. After stirring at room temperature for 4 hours, sodium sulfite was added. The reaction mixture was washed with a saturated sodium bicarbonate solution and water, dried over $MgSO_4$, and concentrated in vacuo. The residue was recrystallized from hexane to give 49.5 g of methyl 5-(3,4-dichlorophenylsulfonyl)-4-(N,N-dipentylcarbamoyl)pentanoate.
Melting point: 52-54°C

| Elemental Analysis (for $C_{23}H_{35}Cl_2NO_5S$): | | | |
|---|---|---|---|
| | C% | H% | N% |
| Calcd. | 54.33 | 6.94 | 2.75 |
| Found | 54.28 | 6.98 | 2.51 |

IR (KBr): $\nu_{C=O}$ 1735, 1635 $cm^{-1}$

$\nu_{SO_2}$ 1325, 1170 $cm^{-1}$

NMR ($CDCl_3$)
δ: 0.88(3H, t, 3=7.1Hz), 0.95(3H, t, J=7.1Hz), 1.15-2.1(14H, m), 2.33(2H, t, J=7.7Hz), 3.0-3.45(6H, m), 3.68-(3H, s), 3.83(1H, dd, J=8.8, 14.3Hz), 7.63(1H, d, J=8.2Hz), 7.72(1H, dd, J=1.7, 8.2Hz), 7.97(1H, d, J=1.7Hz)

Example 2

Methyl 5-(3,4-dichlorophenylsulfonyl)-4-(N,N-dipentylcarbamoyl)pentanoate

Into a solution of methyl 5-(3,4-dichlorophenylsulfinyl)-4-(N,N-dipentylcarbamoyl)pentanoate (95 mg) in dry dichloromethane (10 ml) was added by portions of m-chloroperbenzoic acid (80%, 51 mg) with stirring at 0°C. After stirring at room temperature for 2 hours, sodium sulfite was added. The reaction mixture was washed with a saturated sodium bicarbonate solution and water, dried over $MgSO_4$, and concentrated in vacuo. The residue was recrystallized from hexane to give 100 mg of methyl 5-(3,4-dichlorophenylsulfonyl)-4-(N,N-dipentylcarbamoyl)pentanoate. The physical properties of this compound were identical to that of the compound, which was prepared in example 1.

Example 3

The compounds in the table were prepared in a similar manner to that described in example 1 or 2. The compounds in which no specific rotation shows are in a racemate.

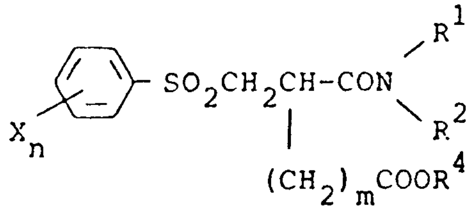

| compound No. | $X_n$ | $R^1$ | $R^2$ | $R^4$ | m | mp (°C) (recryst. solvent) | Specific rotation $[\alpha]_D^{25}$ | IR ($cm^{-1}$) | NMR (δ, $CDCl_3$) |
|---|---|---|---|---|---|---|---|---|---|
| 1 | 3,4-dichloro | pentyl | pentyl | methyl | 2 | the reference to example 1 | | | |
| 2 | 3,4-dichloro | pentyl | pentyl | methyl | 2 | oil | -1.81° (C=1.10, MeOH) | 1735 1640 (C=O) 1320 1160 ($SO_2$) | the agreement with example 1 |
| 3 | 3,4-dichloro | pentyl | pentyl | methyl | 2 | oil | +1.80° (C=1.22, MeOH) | | the agreement with the compound 2 |
| 4 | 3,4-dichloro | 3-methoxy-propyl | pentyl | methyl | 2 | oil | | 1735 1640 (C=O) 1320 1160 ($SO_2$) | 0.88 and 0.94(3H, t, J=7.1Hz), 1.15-2.1(10H, m), 2.33(2H, t, J=7.1 Hz), 3.0-3.5(11H, m), 3.68(3H, s), 3.75-3.95 (1H, m), 7.63 and 7.64 (1H, d, J=8.2Hz), 7.72 and 7.73(1H, dd, J=2.2, 8.2Hz), 7.97(1H, d, J=2.2Hz) |

T-19

| compound No. | $X_n$ | $R^1$ | $R^2$ | $R^4$ | m | mp (°C) (recryst. solvent) | Specific rotation $[\alpha]_D^{25}$ | IR ($cm^{-1}$) | NMR (δ, $CDCl_3$) |
|---|---|---|---|---|---|---|---|---|---|
| 5 | 3,4-dichloro | 3-methoxy-propyl | pentyl | methyl | 2 | oil | +2.21° (C=1.63, MeOH) | | the agreement with the compound 4 |
| 6 | 3,4-dichloro | 3-methoxy-propyl | pentyl | methyl | 2 | oil | -2.13° (C=1.03, MeOH) | | the agreement with the compound 4 |
| 7 | 3,5-dichloro | pentyl | pentyl | methyl | 2 | oil | | 1735 1635 (C=O) 1320 1160 ($SO_2$) | 0.89(3H, t, J=7.1Hz), 0.95(3H, t, J=7.1Hz), 1.15-2.1(14H, m), 2.34 (2H, t, J=7.1Hz), 3.0-3.45(6H, m), 3.68(3H, s), 3.84(1H, dd, J=8.8, 14.3 Hz), 7.61(1H, t, J=1.7 Hz), 7.76(2H, d, J=1.7Hz) |
| 8 | 4-chloro | pentyl | pentyl | methyl | 2 | oil | | 1735 1640 (C=O) 1315 1150 ($SO_2$) | 0.89(3H, t, J=7.1Hz), 0.94(3H, t, J=7.1Hz), 1.15-2.1(14H, m), 2.33 (2H, t, J=7.2Hz), 3.05-3.45(6H, m), 3.67(3H, s), 3.78(1H, dd, J=8.3 13.7Hz), 7.52(2H, dd, J=2.2, 8.2Hz), 7.84(2H, dd, J=2.2, 8.2Hz) |

T-20

| compound No. | $X_n$ | $R^1$ | $R^2$ | $R^4$ | m | mp (°C) (recryst. solvent) | Specific rotation $[\alpha]_D^{25}$ | IR ($cm^{-1}$) | NMR (δ, $CDCl_3$) |
|---|---|---|---|---|---|---|---|---|---|
| 9 | 3-chloro | pentyl | pentyl | methyl | 2 | oil | | 1735 1635 (C=O) 1320 1150 ($SO_2$) | 0.88(3H, t, J=7.1Hz), 0.95(3H, t, J=7.1Hz), 1.15-2.1(14H, m), 2.33 (2H, t, J=7.2Hz), 3.05-3.45(6H, m), 3.68(3H, s), 3.81(1H, dd, J=8.2, 14.3Hz), 7.50(1H, t, J=7.7Hz), 7.55-7.65(1H, m), 7.75-7.85(1H, m), 7.88(1H, t, J=1.7Hz) |
| 10 | 3-bromo | pentyl | pentyl | methyl | 2 | oil | | 1740 1635 (C=O) 1320 1150 ($SO_2$) | 0.88(3H, t, J=7.1Hz), 0.95(3H, t, J=7.1Hz) 1.15-2.1(14H, m), 2.34 (2H, t, J=7.1Hz), 3.0-3.45(6H, m), 3.68(3H, s), 3.81(1H, dd, J=8.2, 13.7Hz), 7.43(1H, t, J=7.7Hz), 7.77(1H, dt, J=7.7, 1.7Hz), 7.84(1H, dt, J=7.7, 1.7Hz), 8.03 (1H, t, J=1.7H) |

T-21

| compound No. | $X_n$ | $R^1$ | $R^2$ | $R^4$ | m | mp (°C) (recryst. solvent) | Specific rotation $[\alpha]_D^{25}$ | IR ($cm^{-1}$) | NMR (δ, $CDCl_3$) |
|---|---|---|---|---|---|---|---|---|---|
| 11 | 3,4-dichloro | pentyl | pentyl | methyl | 1 | oil | | 1735 1640 (C=O) 1320 1155 ($SO_2$) | 0.88(3H, t, J=6.6Hz), 0.94(3H, t, J=6.6Hz) 1.15-1.7(12H, m), 2.74 (2H, d, J=7.2Hz), 3.1-3.35(5H, m), 3.5-3.75 (5H, m), 7.65(1H, d, J=8.2Hz), 7.73(1H, dd, J=2.2, 8.2Hz), 7.99(1H, d, J=2.2Hz) |
| 12 | 3,4-dichloro | pentyl | pentyl | methyl | 3 | oil | | 1735 1640 (C=O) 1320 1150 ($SO_2$) | 0.85-1.0(6H, m), 1.15-1.8(16H, m), 2.25-2.35(2H, m), 3.05-3.4 (6H, m), 3.66(3H, s), 3.85(1H, dd, J=8.8, 14.3 Hz), 7.63(1H, d, J=8.2 Hz), 7.72(1H, dd, J=2.2, 8.2Hz), 7.96(1H, d, J=2.2Hz) |

T-22

| compound No. | $X_n$ | $R^1$ | $R^2$ | $R^4$ | m | mp (°C) (recryst. solvent) | Specific rotation $[\alpha]_D^{25}$ | IR ($cm^{-1}$) | NMR (δ, $CDCl_3$) |
|---|---|---|---|---|---|---|---|---|---|
| 13 | 3,4-dichloro | methyl | butyl | methyl | 2 | oil | | 1735 1640 (C=O) 1320 1160 ($SO_2$) | 0.92 and 0.99(3H, t, J=7.1Hz), 1.2-2.1(6H, m), 2.34(2H, t, J=7.1 Hz), 2.8-3.55(7H, m), 3.69(3H, s), 3.75-3.95 (1H, m), 7.6-7.75(2H, m), 7.96(1H, d, J=2.2Hz) |
| 14 | 3,4-dichloro | butyl | butyl | methyl | 2 | oil | | 1735 1635 (C=O) 1320 1155 ($SO_2$) | 0.91(3H, t, J=7.1Hz), 0.99(3H, t, J=7.1Hz), 1.2-2.1(10H, m), 2.34 (2H, t, J=7.1Hz), 3.0-3.45(6H, m), 3.68(3H, s), 3.84(1H, dd, J=8.8, 14.3Hz), 7.63(1H, d, J=8.2Hz), 7.73(1H, dd, J=2.2, 8.2Hz), 7.97(1H, d, J=2.2Hz) |

T-23

| compound No. | $X_n$ | $R^1$ | $R^2$ | $R^4$ | m | mp (°C) (recryst. solvent) | Specific rotation $[\alpha]_D^{25}$ | IR ($cm^{-1}$) | NMR (δ, $CDCl_3$) |
|---|---|---|---|---|---|---|---|---|---|
| 15 | 3,4-dichloro | hexyl | hexyl | methyl | 2 | oil | | 1735 1640 (C=O) 1320 1160 ($SO_2$) | 0.87(3H, t, J=6.6Hz), 0.92(3H, t, J=6.6Hz), 1.15-2.1(18H, m), 2.33 (2H, t, J=7.1Hz), 3.0-3.45(6H, m), 3.68(3H, s), 3.84(1H, dd, J=8.8, 14.3Hz), 7.63(1H, d, J=8.2Hz), 7.72(1H, dd, J=2.2, 8.2Hz), 7.97(1H, d, J=2.2Hz) |
| 16 | 3,4-dichloro | heptyl | heptyl | methyl | 2 | 50-52 (hexane) | | 1730 1620 (C=O) 1310 1145 ($SO_2$) | 0.8-1.0(6H, m), 1.15-2.1(22H, m), 2.33(2H, t, J=7.2Hz), 3.0-3.45 (6H, m), 3.68(3H, s), 3.84(1H, dd, J=8.8, 14.3 Hz), 7.63(1H, d, J=8.2 Hz), 7.72(1H, dd, J=2.2, 8.2Hz), 7.97(1H, d, J=2.2Hz) |

T-24

| compound No. | $X_n$ | $R^1$ | $R^2$ | $R^4$ | m | mp (°C) (recryst. solvent) | Specific rotation $[\alpha]_D^{25}$ | IR ($cm^{-1}$) | NMR (δ, $CDCl_3$) |
|---|---|---|---|---|---|---|---|---|---|
| 17 | 3,4-dichloro | iso-pentyl | iso-pentyl | methyl | 2 | oil | | 1735 1640 (C=O) 1320 1155 ($SO_2$) | 0.91(6H, d, J=6.6Hz), 0.98(6H, d, J=6.1Hz), 1.25-2.1(8H, m), 2.33 (2H, t, J=7.1Hz), 3.0-3.45(6H, m), 3.68(3H, s), 3.83(1H, dd, J=8.8, 14.3Hz), 7.63(1H, d, J=8.2Hz), 7.73(1H, dd, J=2.2, 8.2Hz), 7.97(1H, d, J=2.2Hz) |
| 18 | 3,4-dichloro | pentyl | pentyl | ethyl | 2 | oil | | 1730 1640 (C=O) 1320 1155 ($SO_2$) | 0.89(3H, t, J=6.6Hz), 0.95(3H, t, J=6.6Hz), 1.15-2.1(17H, m), 2.32 (2H, t, J=7.1Hz), 3.0-3.45(6H, m), 3.84(1H, dd, J=8.8, 14.3Hz), 4.13 (2H, q, J=7.1Hz), 7.63 (1H, d, J=8.2Hz), 7.72 (1H, dd, J=2.2, 8.2Hz), 7.97(1H, d, J=2.2Hz) |

T-25

Example 4

5-(3,4-Dichlorophenylsulfonyl)-4-(N,N-dipentylcarbamoyl)pentanoic acid

To a solution of methyl 5-(3,4-dichlorophenylsulfonyl)-4-(N,N-dipentylcarbamoyl)pentanoate (40.3 g) in ethanol (500 ml) was added a 2N sodium hydroxide solution (40 ml). After stirring at room temperature for 16 hours, the reaction mixture was concentrated in vacuo, acidified with a dilute hydrochloric acid, and extracted with chloroform. The organic layer was washed with water, dried over $MgSO_4$, and evaporated at reduced pressure. The residue was recrystallized from diethyl ether-hexane to give 38.2 g of 5-(3,4-dichlorophenylsulfonyl)-4-(N,N-dipentylcarbamoyl)pentanoic acid.
Melting point: 76-79 °C

| Elemental Analysis (for $C_{22}H_{33}Cl_2NO_5S$): | | | |
|---|---|---|---|
| | C% | H% | N% |
| Calcd. | 53.44 | 6.73 | 2.83 |
| Found | 53.17 | 6.68 | 2.76 |

IR (KBr): $\nu_{C=O}$ 1730, 1605 $cm^{-1}$

$$\nu_{SO_2}\ 1320,\ 1140\ cm^{-1}$$

NMR ($CDCl_3$)
δ: 0.88(3H, t, J=7.1Hz), 0.94(3H, t, J=7.1Hz), 1.15-2.1(14H, m), 2.39(2H, t, J=7.1Hz), 3.0-3.5(6H, m), 3.83-(1H, dd, J=8.8, 14.3Hz), 7.63(1H, d, J=8.2Hz), 7.73(1H, dd, J=2.2, 8.2Hz), 7.97(1H, d, J=2.2Hz)

Example 5

The compounds in the table were prepared in a similar manner to that described in example 4. The compounds in which no specific rotation shows are in a racemate.

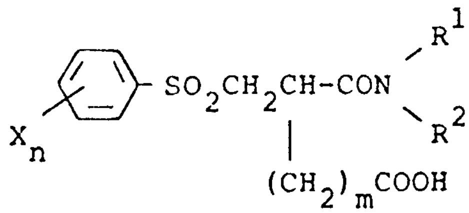

| compound No. | $X_n$ | $R^1$ | $R^2$ | m | mp (°C) (recryst. solvent) | Specific rotation $[\alpha]_D^{25}$ | IR ($cm^{-1}$) | NMR (δ, $CDCl_3$) |
|---|---|---|---|---|---|---|---|---|
| 19 | 3,4-dichloro | pentyl | pentyl | 2 | the reference to example 4 | | | |
| 20 | 3,4-dichloro | pentyl | pentyl | 2 | oil | -6.66° (C=1.20, MeOH) | 1725 1620 (C=O) 1320 1160 ($SO_2$) | the agreement with example 4 |
| 21 | 3,4-dichloro | pentyl | pentyl | 2 | oil | +6.60° (C=1.12, MeOH) | the agreement with the compound 20 | |
| 22 | 3,4-dichloro | 3-methoxy-propyl | pentyl | 2 | 88-91 (iso-propyl ether-hexane) | | 1730 1620 (C=O) 1330 1160 ($SO_2$) | 0.88 and 0.94(3H, t, J=7.1Hz), 1.15-2.1(10H, m), 2.39(2H, t, J=7.1Hz), 3.0-3.55(11H, m), 3.7-3.95(1H, m), 7.6-7.8 (2H, m), 7.97(1H, d, J=2.2Hz) |

T-26

| compound No. | $X_n$ | $R^1$ | $R^2$ | m | mp (°C) (recryst. solvent) | Specific rotation $[\alpha]_D^{25}$ | IR ($cm^{-1}$) | NMR (δ, $CDCl_3$) |
|---|---|---|---|---|---|---|---|---|
| 23 | 3,4-dichloro | 3-methoxy-propyl | pentyl | 2 | oil | -3.67° (C=1.36, MeOH) | 1730 1635 (C=O) 1330 1160 ($SO_2$) | the agreement with the compound 22 |
| 24 | 3,4-dichloro | 3-methoxy-propyl | pentyl | 2 | oil | +3.23° (C=1.88, MeOH) | the agreement with the compound 23 | |
| 25 | 3,5-dichloro | pentyl | pentyl | 2 | 131-132 (diethyl ether) | | 1720 1620 (C=O) 1320 1165 ($SO_2$) | 0.89(3H, t, J=7.1Hz), 0.94(3H, t, J=7.1Hz), 1.15-2.1(14H, m), 2.40(2H, t, J=7.2Hz), 3.0-3.5(6H, m), 3.84(1H, dd, J=8.8, 14.3Hz), 7.61(1H, t, J=2.2Hz), 7.77(2H, d, J=2.2Hz) |
| 26 | 4-chloro | pentyl | pentyl | 2 | oil | | 1730 1635 1610 (C=O) 1315 1150 ($SO_2$) | 0.89(3H, t, J=6.6Hz), 0.93(3H, t, J=6.6Hz), 1.15-2.1(14H, m), 2.38(2H, t, J=7.1Hz), 3.05-3.5(6H, m), 3.77(1H, dd, J=7.7, 13.7Hz), 7.52(2H, d, J=8.8Hz), 7.84(2H, d, J=8.8Hz) |

T-27

| compound No. | $X_n$ | $R^1$ | $R^2$ | m | mp (°C) (recryst. solvent) | Specific rotation $[\alpha]_D^{25}$ | IR ($cm^{-1}$) | NMR (δ, $CDCl_3$) |
|---|---|---|---|---|---|---|---|---|
| 27 | 3-chloro | pentyl | pentyl | 2 | 88-89.5 (diethyl ether-hexane) | | 1730 1630 (C=O) 1320 1135 ($SO_2$) | 0.88(3H, t, J=7.1Hz), 0.94(3H, t, J=7.1Hz), 1.2-2.1(14H, m), 2.39(2H, t, J=7.1Hz), 3.0-3.5(6H, m), 3.79(1H, dd, J=8.2, 13.7Hz), 7.50(1H, t, J=7.7Hz), 7.6-7.65(1H, m), 7.75-7.85(1H, m), 7.88(1H, t, J=1.7Hz) |
| 28 | 3-bromo | pentyl | pentyl | 2 | 93-95 (diethyl ether-hexane) | | 1725 1625 (C=O) 1320 1135 ($SO_2$) | 0.89(3H, t, J=7.1Hz), 0.94(3H, t, J=7.1Hz), 1.15-2.1(14H, m), 2.39(2H, t, J=7.1Hz), 3.0-3.5(6H, m), 3.80(1H, dd, J=8.3, 14.3Hz), 7.44(1H, t, J=7.7Hz), 7.75-7.9(2H, m), 8.03 (1H, t, J=1.7Hz) |
| 29 | 3,4-dichloro | pentyl | pentyl | 1 | 96-99 (hexane) | | 1705 1625 (C=O) 1320 1155 ($SO_2$) | 0.88(3H, t, J=7.1Hz), 0.94(3H, t, J=7.1Hz), 1.15-1.75(12H, m), 2.79(2H, d, J=5.5Hz), 3.15-3.8(7H, m), 7.66(1H, d, J=8.2Hz), 7.75(1H, d, J=8.2Hz), 8.00(1H, s) |

T-28

| compound No. | $X_n$ | $R^1$ | $R^2$ | m | mp (°C) (recryst. solvent) | Specific rotation $[\alpha]_D^{25}$ | IR ($cm^{-1}$) | NMR (δ, $CDCl_3$) |
|---|---|---|---|---|---|---|---|---|
| 30 | 3,4-dichloro | pentyl | pentyl | 3 | 104-106 (diethyl ether-hexane) | | 1705 1635 (C=O) 1320 1150 ($SO_2$) | 0.88(3H, t, J=7.1Hz), 0.95(3H, t, J=7.1Hz), 1.2-1.8(16H, m), 2.25-2.4(2H, m), 3.05-3.35 (6H, m), 3.86(1H, dd, J=8.8, 13.7Hz), 7.63(1H, d, J=8.2Hz), 7.72(1H, dd, J=2.2, 8.2Hz), 7.96(1H, d, J=2.2Hz) |
| 31 | 3,4-dichloro | methyl | butyl | 2 | 112-113 (iso-propyl ether) | | 1740 1640 1610 (C=O) 1320 1135 ($SO_2$) | 0.92 and 0.98(3H, t, J=7.1Hz), 1.2-2.1(6H, m), 2.3-2.6 (2H, m), 2.8-3.6(7H, m), 3.75-3.95(1H, m), 7.6-7.75(2H, m), 7.96(1H, d, J=2.2Hz) |
| 32 | 3,4-dichloro | butyl | butyl | 2 | oil | | 1730 1640 1610 (C=O) 1320 1160 ($SO_2$) | 0.91(3H, t, J=7.1Hz), 0.98(3H, t, J=7.1Hz), 1.2-2.1(10H, m), 2.39(2H, t, J=7.1Hz), 3.0-3.5(6H, m), 3.83(1H, dd, J=8.8, 14.3Hz), 7.63(1H, d, J=8.2Hz), 7.73(1H, dd, J=2.2, 8.2Hz), 7.97(1H, d, J=2.2Hz) |

T-29

| compound No. | $X_n$ | $R^1$ | $R^2$ | m | mp (°C) (recryst. solvent) | Specific rotation $[\alpha]_D^{25}$ | IR ($cm^{-1}$) | NMR (δ, $CDCl_3$) |
|---|---|---|---|---|---|---|---|---|
| 33 | 3,4-dichloro | hexyl | hexyl | 2 | 67-68 (diethyl ether-hexane) | | 1720 1630 (C=O) 1325 1135 ($SO_2$) | 0.8-1.0(6H, m), 1.15-2.1 (18H, m), 2.38(2H, t, J=7.1Hz), 2.95-3.5(6H, m), 3.83 (1H, dd, J=8.2, 14.3Hz), 7.63 (1H, d, J=8.2Hz), 7.73(1H, dd, J=1.7, 8.2Hz), 7.97(1H, d, J=1.7Hz) |
| 34 | 3,4-dichloro | heptyl | heptyl | 2 | 79-81 (iso-propyl ether-hexane) | | 1715 1610 (C=O) 1320 1155 ($SO_2$) | 0.8-1.0(6H, m), 1.1-2.1 (22H m), 2.39(2H, t, J=7.1Hz), 3.0-3.5(6H, m), 3.83 (1H, dd, J=8.8, 14.3Hz), 7.63 (1H, d, J=8.2Hz), 7.72(1H, dd, J=2.2, 8.2Hz), 7.97(1H, d, J=2.2Hz) |
| 35 | 3,4-dichloro | iso-pentyl | iso-pentyl | 2 | oil | | 1720 1635 (C=O) 1320 1155 ($SO_2$) | 0.88(6H, d, J=6.6Hz), 0.94(6H, d, J=6.6Hz), 1.2-2.4(10H, m), 3.0-3.35(6H, m), 3.75-3.95 (1H, m), 7.61(1H, d, J=8.8Hz), 7.74(1H, dd, J=2.2, 8.8Hz), 7.95(1H, d, J=2.2Hz) |

T-30

Example 6

Methyl 4-(N-benzyl-N-methylcarbamoyl)-5-(3,4-dichlorophenylsulfonyl)pentanoate

Into a solution of methyl 4-(N-benzyl-N-methylcarbamoyl)-5-(3,4-dichlorophenylthio)pentanoate (110 mg) in dry chloroform (4 ml) was added by portions of m-chloroperbenzoic acid (80%, 160 mg) with stirring at 0°C. After stirring at room temperature for 4 hours, sodium sulfite was added. The reaction mixture was washed with a saturated sodium bicarbonate solution and water, dried over $MgSO_4$, and concentrated in vacuo. The residue was purified by preparative thin-layer chromatography on silica developing with dichloromethane/diethyl ether (2:1) to give 100 mg of methyl 4-(N-benzyl-N-methylcarbamoyl)-5-(3,4-dichlorophenylsulfonyl)pentanoate as oil.
IR (neat): $\nu_{C=O}$ 1730, 1640 $cm^{-1}$

$$\nu_{SO_2}\ 1315,\ 1155\ cm^{-1}$$

NMR ($CDCl_3$)
δ: 1.75-2.4(4H, m), 2.9-3.15(4H, m), 3.45-3.7(4H, m), 3.84 and 3.94(1H, dd, J=9.3, 13.7Hz), 4.3-4.75(2H, m), 7.2-7.45(5H, m), 7.55-7.75(2H, m), 7.92 and 8.00(1H, d, J=2.2Hz)

Example 7

Methyl 5-(3,4-dichlorophenylsulfonyl)-4-[N,N-bis(3-phenylpropyl)carbamoyl]pentanoate

Into a solution of methyl 5-(3,4-dichlorophenylsulfinyl)-4-[N,N-bis(3-phenylpropyl)carbamoyl]pentanoate (220 mg) in dry dichloromethane (20 ml) was added by portions of m-chloroperbenzoic acid (80%, 120 mg) with stirring at 0°C. After stirring at room temperature for 4 hours, sodium sulfite was added. The reaction mixture was washed with a saturated sodium bicarbonate solution and water, dried over $MgSO_4$, and concentrated in vacuo. The residue was purified by flash column chromatography on silica eluting with chloroform/ethanol (50:1) to give 200 mg of methyl 5-(3,4-dichlorophenylsulfonyl)-4-[N,N-bis(3-phenylpropyl)carbamoyl]pentanoate as oil.
IR (neat): $\nu_{C=O}$ 1735, 1640 $cm^{-1}$

$$\nu_{SO_2}\ 1320,\ 1160\ cm^{-1}$$

NMR ($CDCl_3$)
δ: 1.7-2.35(8H, m), 2.5-2.75(4H, m), 3.0-3.45(6H, m), 3.63(3H, s), 3.84(1H, dd, J=9.3, 14.3Hz), 7.05-7.35-(10H, m), 7.59(1H, d, J=8.2Hz), 7.70(1H, dd, J=2.2, 8.2Hz), 7.96(1H, d, J=2.2Hz)

Example 8

The compounds in the table were prepared in a similar manner to that described in example 6 or 7 (all compounds were oils).

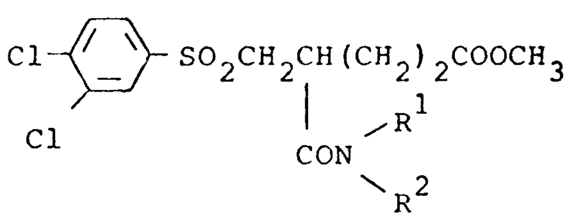

| compound No. | $R^1$ | $R^2$ | IR ($cm^{-1}$) | NMR (δ, $CDCl_3$) |
|---|---|---|---|---|
| 36 | pentyl | phenethyl | 1730, 1635 (C=O) 1315, 1155 ($SO_2$) | 0.89 and 0.93(3H, t, J=7.1Hz), 1.2-2.1(8H, m), 2.30 (2H, t, J=7.1Hz), 2.75-3.85(12H, m), 7.15-7.4(5H, m), 7.63 and 7.64(1H, d, J=8.2Hz), 7.73(1H, dd, J=2.2, 8.2Hz), 7.98 and 7.99(1H, d, J=2.2Hz) |
| 37 | pentyl | 3-phenyl-propyl | 1730, 1635 (C=O) 1315, 1155 ($SO_2$) | 0.8-1.0(3H, m), 1.15-2.1(10H, m), 2.2-2.4(2H, m), 2.55-2.75(2H, m), 3.0-3.45(6H, m), 3.65 and 3.66(3H, s), 3.75-3.9(1H, m), 7.15-7.4(5H, m), 7.55-7.8(2H, m), 7.96(1H, d, J=2.2Hz) |
| 38 | phenethyl | phenethyl | 1725, 1635 (C=O) 1315, 1160 ($SO_2$) | 1.7-2.0(2H, m), 2.15-2.35(2H, m), 2.75-3.0(4H, m), 3.07(1H, dd, J=3.8, 13.7Hz), 3.3-3.75(9H, m), 7.15-7.4(10H, m), 7.63(1H, d, J=8.2Hz), 7.73(1H, dd, J=2.2, 8.2Hz), 7.99(1H, d, J=2.2Hz) |
| 39 | 3-phenyl-propyl | 3-phenyl-propyl | the reference to example 7 | |
| 40 | hexyl | 4-phenyl-butyl | 1735, 1640 (C=O) 1320, 1155 ($SO_2$) | 0.87 and 0.92(3H, t, J=6.6Hz), 1.2-2.1(14H, m), 2.29 and 2.33(2H, t, J=7.1Hz), 2.62 and 2.70(2H, t, J=7.1Hz), 3.0-3.45(6H, m), 3.66 and 3.67(3H, s), 3.83 (1H, dd, J=8.8, 14.3Hz), 7.1-7.35(5H, m), 7.61 and 7.62(1H, d, J=8.2Hz), 7.69 and 7.71(1H, dd, J=2.2, 8.2Hz), 7.95 and 7.97(1H, d, J=2.2Hz) |
| 41 | methyl | benzyl | the reference to example 6 | |

T-31

Example 9

5-(3,4-Dichlorophenylsulfonyl)-4-(N-penthyl-N-phenethylcarbamoyl)pentanoic acid

To a solution of methyl 5-(3,4-dichlorophenylsulfonyl)-4-(N-pentyl-N-phenethylcarbamoyl)pentanoate (57

mg) in ethanol (1 ml) was added a 1N sodium hydroxide solution (0.12 ml). After stirring at room temperature for 16 hours, the reaction mixture was concentrated in vacuo, acidified with a dilute hydrochloric acid, and extracted with chloroform. The organic layer was washed with water, dried over $MgSO_4$, and evaporated at reduced pressure. The residue was purified by preparative thin-layer chromatography on silica developing with chloroform/ethanol (10:1) to give 45 mg of 5-(3,4-dichlorophenylsulfonyl)-4-(N-pentyl-N-phenethylcarbamoyl)pentanoic acid as oil.
IR (neat): $\nu_{C=O}$ 1730, 1630, 1610 $cm^{-1}$

$$\nu_{SO_2}\ 1315,\ 1155\ cm^{-1}$$

NMR ($CDCl_3$)
δ: 0.89 and 0.92(3H, t, J = 7.1Hz), 1.15-2.1(8H, m), 2.31 and 2.34(2H, t, J = 6.6Hz), 2.7-3.85(9H, m), 7.15-7.4-(5H, m), 7.63 and 7.64(1H, d, J = 8.2Hz), 7.73(1H, dd, J = 2.2, 8.2Hz), 7.98 and 7.99(1H, d, J = 2.2Hz)

Example 10

The compounds in the table were prepared in a similar manner to that described in example 9.

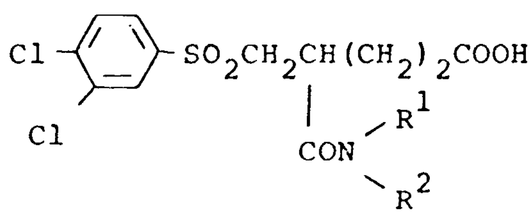

| compound No. | $R^1$ | $R^2$ | mp (°C) (recryst. solvent) | IR ($cm^{-1}$) | NMR (δ, $CDCl_3$) |
|---|---|---|---|---|---|
| 42 | pentyl | phenethyl | the reference to example 9 | | |
| 43 | pentyl | 3-phenyl-propyl | oil | 1725, 1630 (C=O) 1315, 1155 ($SO_2$) | 0.87 and 0.91(3H, t, J=7.1Hz), 1.1-2.15 (10H, m), 2.2-2.45(2H, m), 2.5-2.75(2H, m), 2.95-3.55(6H, m), 3.65-3.9(1H, m), 7.1-7.4(5H, m), 7.55-7.75(2H, m), 7.96 (1H, d, J=1.7Hz) |
| 44 | phenethyl | phenethyl | oil | 1730, 1640 (C=O) 1320, 1160 ($SO_2$) | 1.7-2.0(2H, m), 2.1-2.4(2H, m), 2.75-3.0(4H, m), 3.06(1H, dd, J=3.8, 13.7Hz), 3.3-3.75(6H, m), 7.15-7.4(10H, m), 7.63(1H, d, J=8.2Hz), 7.73(1H, dd, J=2.2, 8.2Hz), 7.99(1H, d, J=2.2Hz) |
| 45 | 3-phenyl-propyl | 3-phenyl-propyl | 114-115 (diethyl ether-hexane) | 1710, 1625 (C=O) 1320, 1160 ($SO_2$) | 1.65-2.4(8H, m), 2.5-2.75(4H, m), 3.03 (1H, dd, J=3.3, 13.7Hz), 3.1-3.4(5H, m), 3.83(1H, dd, J=8.8, 13.7Hz), 7.05-7.4(10H, m), 7.59(1H, d, J=8.2Hz), 7.69(1H, dd, J=2.2, 8.2Hz), 7.95(1H, d, J=2.2Hz) |
| 46 | hexyl | 4-phenyl-butyl | oil | 1730, 1635 (C=O) 1320, 1155 ($SO_2$) | 0.87 and 0.91(3H, t, J=6.6Hz), 1.15-2.1 (14H, m), 2.32 and 2.37(2H, t, J=7.1Hz), 2.55-2.75(2H, m), 3.0-3.5(6H, m), 3.7-3.9(1H, m), 7.1-7.35(5H, m), 7.61 and 7.62 (1H, d, J=8.2Hz), 7.70 and 7.71(1H, dd, J=2.2, 8.2Hz), 7.95 and 7.96(1H, d, J=2.2Hz) |

T-32

| compound No. | $R^1$ | $R^2$ | mp (°C) (recryst. solvent) | IR ($cm^{-1}$) | NMR (δ, $CDCl_3$) |
|---|---|---|---|---|---|
| 47 | methyl | benzyl | 110-112 (iso-propyl ether) | 1710, 1640 (C=O) 1320, 1145 ($SO_2$) | 1.6-2.45(4H, m), 2.9-3.2(4H, m), 3.4-4.0(2H, m), 4.3-4.75(2H, m), 7.15-7.45 (5H, m), 7.55-7.8(2H, m), 7.92 and 8.00 (1H, d, J=1.7Hz) |

T-33

Example 11

Methyl 5-(4-acetylphenylsulfonyl)-4-(N,N-dipentylcarbamoyl)pentanoate

Into a solution of methyl 5-(4-acetylphenylthio)-4-(N,N-dipentylcarbamoyl)pentanoate (0.44 g) in dry

dichloromethane (10 ml) was added by portions of m-chloroperbenzoic acid (80%, 0.52 g) with stirring at 0°C. After stirring at room temperature for 2 hours, sodium sulfite was added. The reaction mixture was washed with a saturated sodium bicarbonate solution and water, dried over $MgSO_4$, and concentrated in vacuo. The residue was purified by flash column chromatography on silica eluting with chloroform/ethanol (10:1) to give 0.45 g of methyl 5-(4-acetylphenylsulfonyl)-4-(N,N-dipentylcarbamoyl)pentanoate as oil.
IR (neat): $\nu_{C=O}$ 1735, 1690, 1635 $cm^{-1}$

$$\nu_{SO_2}\ 1320,\ 1150\ cm^{-1}$$

NMR ($CDCl_3$)
δ: 0.88(3H, t, J=7.1Hz), 0.94(3H, t, J=7.1Hz), 1.15-2.1(14H, m), 2.33(2H, t, J=7.1Hz), 2.66(3H, s), 3.0-3.5-(6H, m), 3.67(3H, s), 3.82(1H, dd, J=8.2, 14.3Hz), 8.00(2H, d, J=8.2Hz), 8.10(2H, d, J=8.2Hz)

Example 12

Methyl 4-(N,N-dipentylcarbamoyl)-5-(4-methylphenylsulfonyl)pentanoate

Into a solution of methyl 4-(N,N-dipentylcarbamoyl)-5-(4-methylphenylsulfinyl)pentanoate (0.15 g) in dry dichloromethane (5 ml) was added by portions of m-chloroperbenzoic acid (80%, 0.09 g) with stirring at 0°C. After stirring at room temperature for 4 hours, sodium sulfite was added. The reaction mixture was washed with a saturated sodium bicarbonate solution and water, dried over $MgSO_4$, and concentrated in vacuo. The residue was purified by flash column chromatography on silica eluting with dichloromethane/diethyl ether (2:1) to give 0.13 g of methyl 4-(N,N-dipentylcarbamoyl)-5-(4-methylphenylsulfonyl)pentanoate as oil.
IR (neat): $\nu_{C=O}$ 1735, 1635 $cm^{-1}$

$$\nu_{SO_2}\ 1315,\ 1150\ cm^{-1}$$

NMR ($CDCl_3$)
δ: 0.88(3H, t, J=7.1Hz), 0.94(3H, t, J=7.1Hz), 1.15-2.1(14H, m), 2.32(2H, t, J=7.1Hz), 2.44(3H, s), 3.0-3.45-(6H, m), 3.66(3H, s), 3.71(1H, dd, J=7.1, 13.7Hz), 7.34(2H, d, J=8.2Hz), 7.78(2H, d, J=8.2Hz)

Example 13

The compounds in the table were prepared in a similar manner to that described in example 11 or 12 (all compounds were oils).

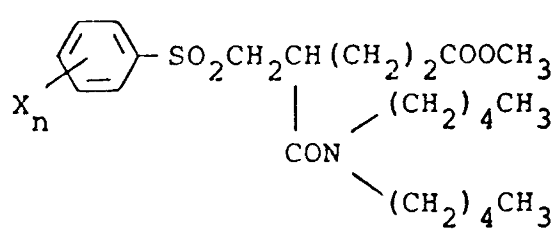

| Xn | IR ($cm^{-1}$) | NMR (δ, $CDCl_3$) |
|---|---|---|
| 3,4-dimethyl | 1730, 1635 (C=O) 1310, 1140 ($SO_2$) | 0.89(3H, t, J=7.1Hz), 0.94(3H, t, J=7.1Hz), 1.15-1.75(12H, m), 1.85-2.1(2H, m), 2.2-2.4(8H, m), 3.05(1H, dd, J=4.4, 13.7Hz), 3.1-3.45(5H, m), 3.6-3.75 (4H, m), 7.29(1H, d, J=8.2Hz), 7.6-7.7(2H, m) |
| 4-nitro | 1730, 1635 (C=O) 1530 ($NO_2$) 1310, 1155 ($SO_2$) | 0.88(3H, t, J=7.1Hz), 0.95(3H, t, J=7.1Hz), 1.15-2.1(14H, m), 2.34(2H, t, J=7.1Hz), 3.0-3.5(6H, m), 3.68(3H, s), 3.90(1H, dd, J=8.8, 13.7Hz), 8.10(2H, d, J=8.8Hz), 8.39(2H, d, J=8.8Hz) |

T - 34

Example 14

5-(4-Acetylphenylsulfonyl)-4-(N,N-dipentylcarbamoyl)pentanoic acid

To a solution of methyl 5-(4-acetylphenylsulfonyl)-4-(N,N-dipentylcarbamoyl)pentanoate (0.35 g) in methanol (15 ml) was added a 1N sodium hydroxide solution (1 ml). After stirring at room temperature for 18 hours, the reaction mixture was concentrated in vacuo, acidified with a dilute hydrochloric acid, and extracted with chloroform. The organic layer was washed with water, dried over $MgSO_4$, and evaporated at reduced pressure. The residue was recrystallized from dichloromethane-hexane to give 0.27 g of 5-(4-acetylphenylsulfonyl)-4-(N,N-dipentylcarbamoyl)pentanoic acid.
Melting point: 69-71 °C

| Elemental Analysis (for $C_{24}H_{37}NO_6S$): | | | |
|---|---|---|---|
| | C% | H% | N% |
| Calcd. | 61.64 | 7.98 | 3.00 |
| Found | 61.37 | 7.76 | 2.92 |

IR (KBr): $\nu_{C=O}$ 1720, 1680, 1610 $cm^{-1}$

$$\nu_{SO_2}\ 1310,\ 1140\ cm^{-1}$$

NMR($CDCl_3$)
δ: 0.88(3H, t, J=7.1Hz), 0.94(3H, t, J=7.1Hz), 1.15-2.1(14H, m), 2.39(2H, t, J=7.1Hz), 2.66(3H, s), 3.05-3.5-(6H, m), 3.81(1H, dd, J=8.2, 14.3Hz), 8.00(2H, d, J=8.2Hz), 8.10(2H, d, J=8.2Hz)

Example 15

The compounds in the table were prepared in a similar manner to that described in example 14.

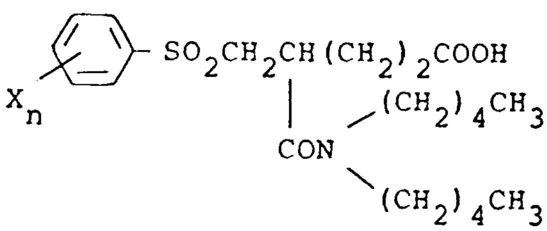

| compound No. | $X_n$ | mp (°C) (recryst. solvent) | IR ($cm^{-1}$) | NMR (δ, $CDCl_3$) |
|---|---|---|---|---|
| 48 | 3,4-dimethyl | 77-79 (chloro-form-hexane) | 1710, 1600 (C=O) 1305, 1150 ($SO_2$) | 0.89(3H, t, J=7.1Hz), 0.93(3H, t, J=7.1Hz), 1.1-2.4(22H, m), 3.06(1H, dd, J=4.4, 13.7Hz), 3.1-3.5 (5H, m), 3.67(1H, dd, J=7.7, 13.7Hz), 7.29(1H, d, J=8.2Hz), 7.55-7.7(2H, m) |
| 49 | 4-methyl | 125-127 (dichloro-methane-hexane) | 1735, 1640 (C=O) 1320, 1155 ($SO_2$) | 0.88(3H, t, J=7.1Hz), 0.93(3H, t, J=7.1Hz), 1.2-2.1(14H, m), 2.38(2H, t, J=7.2Hz), 2.44(3H, s), 3.0-3.5(6H, m), 3.69(1H, dd, J=7.1, 13.7Hz), 7.34(2H, d, J=8.2Hz), 7.78(2H, d, J=8.2Hz) |
| 50 | 4-nitro | 88-90 (diethyl-ether-hexane) | 1710, 1605 (C=O) 1535 ($NO_2$) 1305, 1150 ($SO_2$) | 0.88(3H, t, J=6.6Hz), 0.94(3H, t, J=6.6Hz), 1.1-2.1(14H, m), 2.40(2H, t, J=7.1Hz), 3.0-3.55(6H, m), 3.89(1H, dd, J=8.8, 14.3Hz), 8.10(2H, d, J=8.8Hz), 8.39(2H, d, J=8.8Hz) |
| 51 | 4-acetyl | the reference to example 14 | | |

T-35

Test example 1

CCK-receptor binding assay in pancreas

Pancreatic plasma membranes were prepared by the method of Chang et al. (Molecular Pharmacology, Vol. 30, p. 212-, 1986). Male Wistar rats were sacrificed by decapitation and the pancreas was removed. The pancreas was dissected free from adipose tissue and minced in 50 volumes ice-cold Tris-HCl buffer (50 mM, pH 7.4 at 37° C). Homogenization was performed using ultra disperser and the homogenate was centrifuged at 50,000 x g for 10 minutes. The pellet was washed by resuspension in the same volume of fresh Tris-HCl buffer and again centrifuged as above. The final pellet was suspended in assay buffer (50 mM Tris-HCl, 5 mM $MgCl_2$, 5 mM dithiothreitol, 2 mg/ml of bovine serum albumin, and 0.14 mg/ml of bacitracin), and used as receptor sources for binding assay.

Thirty pM $^{125}$I-CCK-8 (final concentration), test drug or vehicle (for total binding) or $10^{-6}$ M CCK-8 (for nonspecific binding) were added to the assay medium containing pancreatic plasma membranes (0.5 mg of original wet weight/ml). After incubation at 37° C for 30 minutes, the assay medium was filtrated under reduced pressure through glass fiber filter and washed four times with 4 ml of ice-cold Tris-HCl buffer. The radioactivity trapped on the filter was counted by $\gamma$-counter (Packard 5650).

Specific binding was defined as the radioactivity bound after subtracting nonspecific binding determined in the presense of $10^{-6}$ M unlabeled CCK-8. $IC_{50}$ value was calculated from inhibition percentage of specific binding by each test drug.

Table 1

| Compound No. | $IC_{50}$ (μM) |
|---|---|
| 1 | 0.88 |
| 11 | 0.98 |
| 12 | 4.8 |
| 18 | 1.1 |
| 19 | 0.20 |
| 20 | 0.11 |
| 21 | 1.1 |
| 22 | 0.23 |
| 23 | 0.29 |
| 24 | 4.0 |
| 25 | 1.5 |
| 26 | 1.0 |
| 27 | 4.7 |

| Compound No. | $IC_{50}$ (μM) |
|---|---|
| 28 | 3.0 |
| 29 | 0.095 |
| 30 | 0.23 |
| 31 | 8.8 |
| 32 | 0.28 |
| 33 | 0.059 |
| 34 | 0.43 |
| 35 | 0.13 |
| 40 | 5.8 |
| 41 | 3.5 |
| 42 | 0.21 |
| 43 | 0.11 |
| 44 | 0.23 |
| 45 | 1.0 |
| 46 | 0.57 |
| 47 | 1.8 |
| 48 | 0.29 |
| 49 | 3.4 |
| 50 | 0.73 |
| 51 | 2.0 |

Test example 2

CCK-antagonism in isolated gallbladder

Gallbladder strips isolated from male Hartley guinea-pig were prepared and suspended in Magnus bath filled with Krebs solution (1 g of initial tension). The solution kept at 37°C was aerated with a mixture of 95% $O_2$ and 5% $CO_2$. The isometric contraction of strips was recorded through a force-displacement transducer. Antagonism by test drugs against gallbladder contraction induced by $10^{-8}$ M CCK-8 was examined and $IC_{50}$ value was calculated.

Table 2

| Compound No. | $IC_{50}$ (μM) |
|---|---|
| 19 | 11 |
| 20 | 5.1 |
| 22 | 3.4 |
| 29 | 37 |

Test example 3

Effect on amylase secretion in rats

Male Wistar rats were anesthetized with urethane (1.5 g/kg, S.C.), and tracheal cannula was inserted. Biliopancreatic juice was collected from the cannula which had been inserted into the common bile duct. Thirty minutes after intraduodenal administration of test drug, anylase secretion was stimulated by subcutaneous injection of CCK-8 (10 μg/kg). Amylase concentration in the sample collected every 30 minutes during experiment was assayed using Amylase B Test (Wako), and $ED_{50}$ value was calculated.

Table 3

| Compound No. | $ED_{50}$ (mg/kg) |
|---|---|
| 19 | 2.2 |
| 20 | 4.7 |
| 22 | 9.5 |
| 29 | 9.9 |
| 33 | 7.1 |

## Claims

1. A compound represented by the formula:

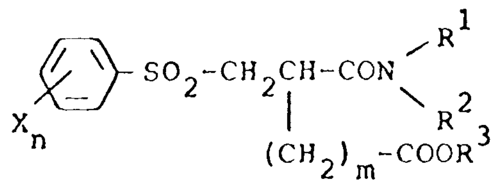

(I)

wherein $R^1$ represents an alkyl group having 1 to 10 carbon atoms, an alkoxyalkyl group having 3 to 7 carbon atoms or an aralkyl group having 7 to 12 carbon atoms; $R^2$ represents an alkyl group having 1 to 10 carbon atoms or an aralkyl group having 7 to 12 carbon atoms; $R^3$ represents a hydrogen atom or an alkyl group having 1 to 4 carbon atoms; X represents a halogen atom, an alkyl group having 1 to 3 carbon atoms, a nitro group or an acetyl group; m is an integer of from 1 to 3; n is 1 or 2; and pharmaceutically acceptable salts thereof.

2. A compound as claimed in claim 1 wherein X is a halogen atom.

3. A compound as claimed in claim 1 or claim 2 wherein m has a value of 2.

4. A compound as claimed in any one of claims 1 to 3 wherein $R^2$ is an alkyl group having 1 to 10 carbon atoms.

**5.** A compound as claimed in any one of claims 1 to 4 wherein $R^1$ is an alkoxyalkyl group having 3 to 7 carbon atoms.

**6.** A compound as claimed in any one of claims 1 to 5 wherein $R^3$ is a hydrogen atom.

**7.** A compound represented by the formula:

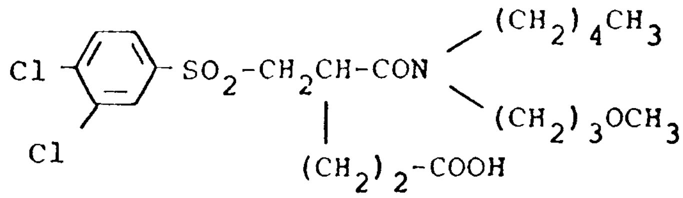

and pharmaceutically acceptable salts thereof.

**8.** A pharmaceutical composition containing as an active ingredient a phenylsulfonylalkanoic acid compound or a pharmaceutically acceptable salt thereof as represented in claim 1.

**9.** A compound represented by the formula:

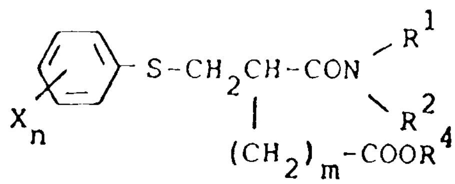

(III)

wherein $R^1$ represents an alkyl group having 1 to 10 carbon atoms, an alkoxyalkyl group having 3 to 7 carbon atoms or an aralkyl group having 7 to 12 carbon atoms; $R^2$ represents an alkyl group having 1 to 10 carbon atoms or an aralkyl group having 7 to 12 carbon atoms; $R^4$ represents an alkyl group having 1 to 4 carbon atoms; X represents a halogen atom, an alkyl group having 1 to 3 carbon atoms, a nitro group or an acetyl group; m is an integer of from 1 to 3; n is 1 or 2.

**10.** A compound represented by the formula:

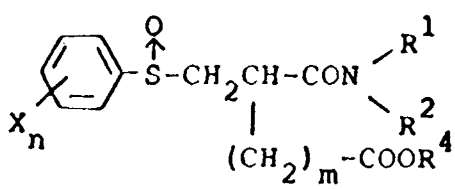

(IV)

wherein $R^1$ represents an alkyl group having 1 to 10 carbon atoms, an alkoxyalkyl group having 3 to 7 carbon atoms or an aralkyl group having 7 to 12 carbon atoms; $R^2$ represents an alkyl group having 1 to 10 carbon atoms or an aralkyl group having 7 to 12 carbon atoms; $R^4$ represents an alkyl group having 1 to 4 carbon atoms; X represents a halogen atom, an alkyl group having 1 to 3 carbon atoms, a nitro group or an acetyl group; m is an integer of from 1 to 3; n is 1 or 2.

**Claims for the following Contracting State : ES**

**1.** A process which comprises oxidising a phenylthioalkanoic acid compound represented by the formula:

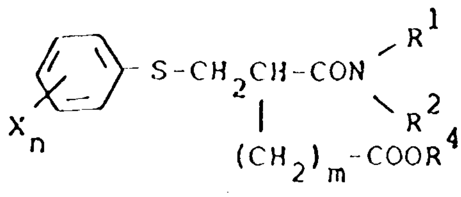

(III)

wherein $R^1$ represents an alkyl group having 1 to 10 carbon atoms, an alkoxyalkyl group having 3 to 7 carbon atoms or an aralkyl group having 7 to 12 carbon atoms; $R^2$ represents an alkyl group having 1 to 10 carbon atoms or an aralkyl group having 7 to 12 carbon atoms; $R^4$ represents an alkyl group having 1 to 4 carbon atoms; X represents a halogen atom, an alkyl group having 1 to 3 carbon atoms, a nitro group or an acetyl group; m is an integer of from 1 to 3; n is 1 or 2, or a phenysulfinylalkanoic acid compound represented by the formula:

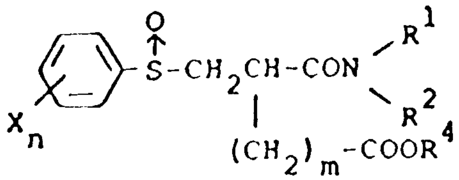

(IV)

wherein $R^1$, $R^2$, $R^4$, X, m and n are as previously defined, to produce a phenylsulfonylalkanoic acid compound represented by the formula:

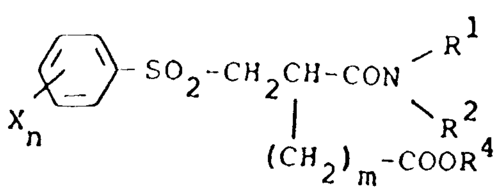

(IV)

in which $R^1$, $R^2$, $R^4$, X, m and n are as previously defined.

2. A process as claimed in claim 1 in which the oxidation is carried out by dissolving the compound of formula (III) in a suitable organic solvent and then adding a molar excess of oxidising agent, cooling and stirring the reaction mixture and then recovering the compound of formula (IV).

3. A process as claimed in claim 2 in which the organic solvent is dichloromethane and the oxidising agent is m-chloroperbenzoic acid.

4. A process as claimed in any one of claims 1 to 3 which includes the further step of hydrolysis of the ester represented by formula (IV) to produce a compound represented by formula (IV) in which $R^1$, $R^2$, X, m and n are as previously defined but $R^4$ is a hydrogen atom.

5. A process as claimed in any one of claims 1 to 4 in which the compound of formula (III) has been prepared by a process involving the steps of (a) reacting a thiophenol compound represented by the formula:

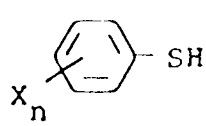

(V)

where X and n are as previously defined with a compound represented by the formula:

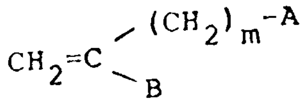 (VI)

where (1) A and B are the same and are a cyano group or an alkoxycarbonyl group having 2 to 5 carbon atoms, or (2) A represents an alkoxycarbonyl group having 2 to 5 carbon atoms and B represents a carboxy group or alkali metal salts thereof, and m is as previously defined, in the presence of a Lewis-base or Lewis-acid to form a product represented by the formula:

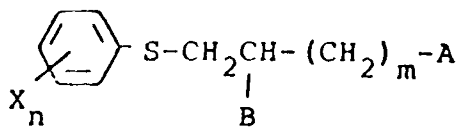 (VII)

wherein A, B, X, m, and n are as previously defined, (b) hydrolyzing and then mono-esterifying the product of (a) to provide a compound represented by the formula:

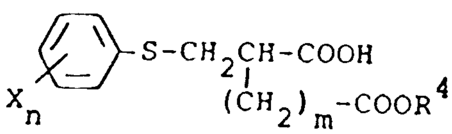 (VIII)

where $R^4$, X, m and n are as previously defined, and then (c) reacting the product of (b) or a reactive functional derivative thereof with an amino compound represented by the formula:

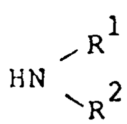 (IX)

wherein $R^1$ and $R^2$ are as previously defined.

European Patent Office

# EUROPEAN SEARCH REPORT

Application Number

EP 91 30 3079

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| E | EP-A-0 433 064 (KISSEI) 19 June 1991<br>*entire document * | 1-10 | C07C317/44<br>A61K31/16<br>C07C323/62 |
| E | PATENT ABSTRACTS OF JAPAN<br>vol. 15, no. 373 (C-869)(4901) 19 September 1991<br>& JP-A-3 148 251 ( KISSEI ) 25 June 1991<br>* abstract * | 1-11 | |
| E | PATENT ABSTRACTS OF JAPAN<br>vol. 15, no. 341 (C-863)(4869) 29 August 1991<br>& JP-A-3 133 952 ( KISSEI ) 7 June 1991<br>* abstract * | 1-11 | |
| E | PATENT ABSTRACTS OF JAPAN<br>vol. 15, no. 377 (C-870)(4905) 24 September 1991<br>& JP-A-3 151 355 ( KISSEI ) 27 June 1991<br>* abstract * | 1-11 | |
| A | BE-A-558 647 (CILAG)<br>* claim 1 * | 1 | |
| | | | TECHNICAL FIELDS SEARCHED (Int. Cl.5 ) |
| | | | C07C |

The present search report has been drawn up for all claims

| Place of search | Date of completion of the search | Examiner |
|---|---|---|
| THE HAGUE | 28 NOVEMBER 1991 | WELLS A.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)